# EUROPEAN PATENT SPECIFICATION

(11) **EP 2 467 398 B1**
(45) Date of publication and mention of the grant of the patent: **09.10.2019**
(21) Application number: 10778750.9
(22) Date of filing: 20.08.2010
(51) Int. Cl.: A61K 31/18, C07K 14/705, A61P 25/04, C12N 15/113

(54) **TRPC4 INHIBITORS AND USES THEREOF**
TRPC4-HEMMER UND IHRE VERWENDUNG
INHIBITEURS DE TRPC4 ET LEURS UTILISATIONS

(30) Priority: 20.08.2009 US 235534 P; 01.10.2009 US 247612 P
(43) Date of publication of application: 27.06.2012
(73) Proprietor: Poseida Therapeutics, Inc., San Diego, California 92121 (US)
(72) Inventor: CRAWFORD, John, Stuart, Lexington, KY 40517 (US); OSTERTAG, Eric, Lexington, KY 40508 (US)
(74) Representative: Cooley (UK) LLP
(86) International application number: PCT/US2010/046151
(87) International publication number: WO 2011/022638

(56) References cited:
- WO-A2-03/057843
- WO-A2-2004/071413
- WO-A2-2005/089206
- ULLOA A ET AL: "Reduction in TRPC4 expression specifically attenuates G-protein coupled receptor-stimulated increases in intracellular calcium in human myometrial cells", CELL CALCIUM (EDINBURGH), vol. 46, no. 1, 1 July 2009 (2009-07-01), pages 73-84, XP026218079, ISSN: 0143-4160
- TIM D PLANT ET AL: "Receptor-operated cation channels formed by TRPC4 and TRPC5", NAUNYN-SCHMIEDEBERG'S ARCHIVES OF PHARMACOLOGY, vol. 371, no. 4, 1 April 2005 (2005-04-01) , pages 266-276, XP019326053, ISSN: 1432-1912
- JEON J P ET AL: "The specific activation of TRPC4 by Gi protein subtype", BIOCHEMICAL AND BIOPHYSICAL RESEARCH COMMUNICATIONS, vol. 377, no. 2, 12 December 2008 (2008-12-12), pages 538-543, XP025646112, ISSN: 0006-291X cited in the application
- XIE Q ET AL: "Expression and functional evaluation of transient receptor potential channel 4 in bovine corneal endothelial cells", EXPERIMENTAL EYE RESEARCH, vol. 81, no. 1, 1 July 2005 (2005-07-01), pages 5-14, XP004939246, ISSN: 0014-4835
- SUNG TAE SIK ET AL: "Functional characteristics of TRPC4 channels expressed in HEK 293 cells", MOLECULES AND CELLS, vol. 27, no. 2, February 2009 (2009-02), pages 167-173, XP002619283, ISSN: 1016-8478 cited in the application
- WU DONGSHENG ET AL: "TRPC4 in rat dorsal root ganglion neurons is increased after nerve injury and is necessary for neurite outgrowth", JOURNAL OF BIOLOGICAL CHEMISTRY, vol. 283, no. 1, January 2008 (2008-01), pages 416-426, XP002619284, ISSN: 0021-9258
- OTSUGURO KEN-ICHI ET AL: "Isoform-specific inhibition of TRPC4 channel by phosphatidylinositol 4,5-bisphosphate.", THE JOURNAL OF BIOLOGICAL CHEMISTRY, vol. 283, no. 15, 11 April 2008 (2008-04-11), pages 10026-10036, XP002619285, ISSN: 0021-9258
- FREICHEL M ET AL: "Functional role of TRPC proteins in vivo: lessons from TRPC-deficient mouse models", BIOCHEMICAL AND BIOPHYSICAL RESEARCH COMMUNICATIONS, vol. 322, no. 4, 1 October 2004 (2004-10-01), pages 1352-1358, XP004536620, ISSN: 0006-291X
- MENENDEZ ET AL: "Calmodulin inhibitors induce spinal analgesia in rats", BRAIN RESEARCH, vol. 731, no. 1-2, 26 August 1996 (1996-08-26), pages 114-121, XP022260476, ISSN: 0006-8993, DOI: 10.1016/0006-8993(96)00480-5
- ZOLTÁN OLÁH ET AL: "Anti-calmodulins and Tricyclic Adjuvants in Pain Therapy Block the TRPV1 Channel", PLOS ONE, vol. 2, no. 6, 20 June 2007 (2007-06-20), page e545, XP055314577, DOI: 10.1371/journal.pone.0000545

## Description

### Field and Background of Invention

The present disclosure, relates to methods including compounds, derivatives, antibodies, interfering RNA, biologies, polypeptides, dominant negative effectors, and their use in the treatment of neuropathic pain by inhibition of transient receptor potential channel, TRPC4.

Mammalian transient receptor potential (TRP) channels are described as six-transmembrane (6-TM) cation-permeable channels. TRP channels control the gating of voltage-dependent Ca2+, K+, and Cl-, and are characterized as calcium-permeable channels with polymodal activation properties. TRP protein structure is thought to be a channel forming structure composed of six transmembrane (TM) domains with a pore domain (P) located between the fifth (S5) and sixth (S6) TMs. TRP channels are activated by three major mechanisms; receptor, ligand and environment direct activation. Receptor activation is carried out by G protein coupled receptors (GCPRs) and tyrosine kinases in three modes which result in liberation of Ca2+ from intracellular stores: hydrolysis of phosphatidylinositol (4,5) bisphosphate (PIP2), diacylglycerol (DAG) and inositol (1,4,5) triphosphate (IP3). Ligand activation occurs by exogenous small molecules (capsaicin, icilin, 2-APB), endogenous lipids or metabolism products (diacylglycerols), purine nucleotides and metabolites (ADP-ribose), and inorganic ions. TRP channels are also activated by environmental triggers such as ambient temperature.

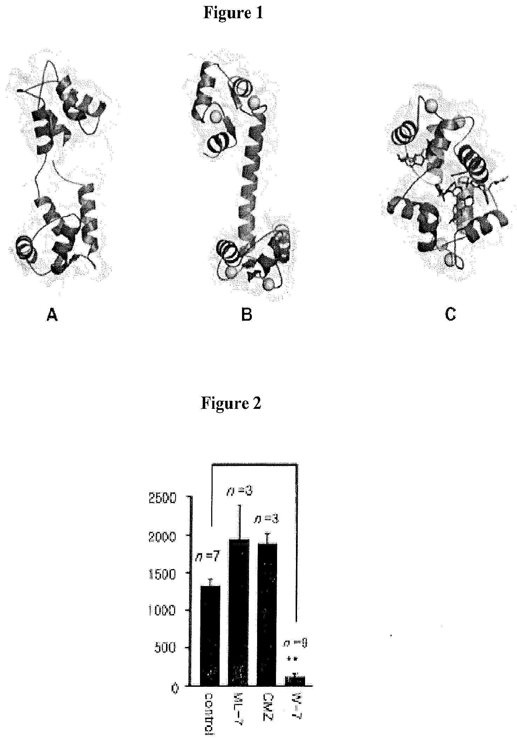

The TRPC subfamily was established by the identification of the first mammalian TRP, TRPC1. Common TRPC motifs are composed of 2-3 ankyrin-like domain repeats and a coiled-coil domain in the N-terminal, followed by six transmembrane domains, the C-term TRP box and a calmodulin (CaM) binding site. The CaM domain in TRPC4 is a calcium binding domain which resembles the CaM protein, which is normally small (∼140+ amino acid in length) dumbbell-shaped composed of two structurally similar globular domains separated by a flexible hinge central helix. The globular domains are homologous and contain pairs of Ca2+ binding helix-loop-helix motifs which are referred to as the EF hand motifs. The typical mechanism of calcium binding occurs at these EF hands, which are composed of two α-helices linked to a 12-residue loop. The EF hand domains become exposed to effectors and targets by protein conformational change. The exposed hydrophobic regions in turn bind basic amphiphilic helices (BAA helices). The hinge of CaM allows for the proteins harboring a CaM domain to contact and activate targets (Figure 1). CaM is highly conserved in animals and plants and acts on many targets including ion channels.

TRPC5 (Accession No. NC_005120.2) is expressed homomerically and also heteromerically complexes with TRPC4 (Accession No. NC_005101.2). TRPC4 and TRPC5 are highly homologous, and are highly expressed in the human brain, uterus, ovary and kidney cells. TRPC4 is a nonselective cation channel which is uniquely activated by Gq/11 family GPCRs through activation of PLCβ, and receptor kinases and receptor tyrosine kinases. Although studies using TRPC4 have shown that activation requires phospholipase C (PLC) activity, neither IP3 nor DAG is sufficient to activate TRPC4. TRPC4 contains a PDZ-binding motif. PDZ domains are common structural motifs which aid proteins in signaling and anchoring transmembrane proteins to the cytoskeleton. PDZ domain scaffolding proteins, as well as signaling molecules, co-immunoprecipitate with TRPC4. PDZ domain is a common structural domain of 80-90 amino-acids found in the signaling proteins of bacteria, yeast, plants, viruses[1] and animals.[2] PDZ is an acronym combining the first letters of three proteins -- post synaptic density protein (PSD95), Drosophila disc large tumor suppressor (DlgA), and zonula occludens-1 protein (zo-1) -- which were first discovered to share the domain. PDZ domains are also referred to as DHR (Dlg homologous region) or GLGF (glycine-leucine-glycine-phenylalanine) domains. These domains help anchor transmembrane proteins to the cytoskeleton and hold together signaling complexes.

Almost every cell type scrutinized contains at least one TRP channel. This large family of physiological important channels has been implicated in many human diseases. Most of the TRP channels are conserved in mice, rats, and humans. Knockout mice studies have proven to be insightful for determining TRP channel functions. *Trpc2* deficient mice are unable to distinguish male from female counterparts and *TRPV6* is upregulated in prostate cancer. TRPC4 transcripts and protein are expressed in primary cultured mouse vascular endothelial cells (MAECs) and the channels can be activated by store-depleted protocols in MAECs. In *Trpc4* deficient mice, agonist induced Ca2+ entry is significantly reduced. *Trpc4-*/*-* mice exhibit significant decrease in endothelium-dependent vasorelaxation in the blood vessels. The *Trpc4* deficient mice display decreased microvascular permeability, and has altered GABA transmitter release from thalamic interneurons. Although *TRPC4* is expressed in the nervous system it has not been validated previously as a target for neuropathic pain and there were no known specific inhibitors for the channel.

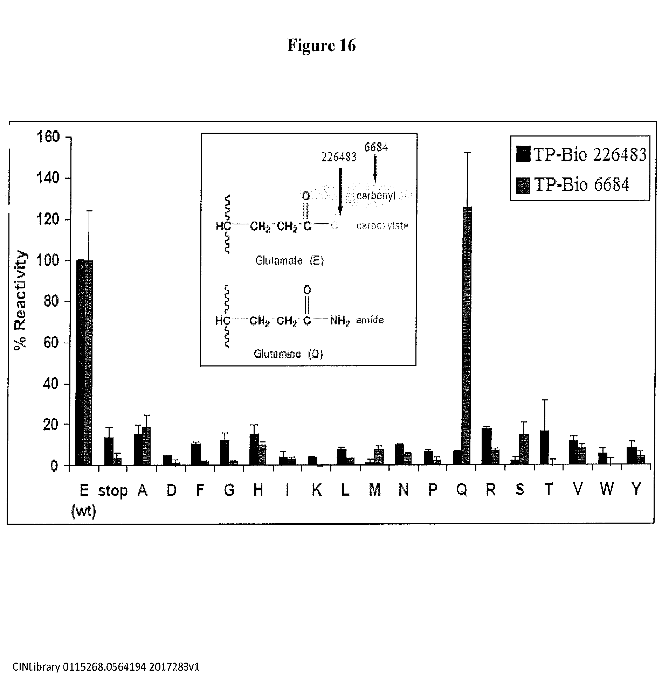

Since the *TRPC4* gene contains an important calmodulin (CaM) it is logical that a CaM inhibitor may have an antagonistic effect on TRPC4 protein function. The best known and most well studied CaM antagonists are analogs of trifluoroperazine (TFP), a phenothiazine derivative. TFP binds to CaM at the hydrophobic pockets of CaM (Figure 1C). Arylalkylamine derivates such as fendiline, and bisindole drug analogs exhibit CaM antagonist activity. Bisindole derivatives vinblastine, navelbine, and an analog termed KAR-2 show extensive CaM inhibitory action. Other well known inhibitors are derived naturally from fungus such as *Emericella* sp, and *Malbranchea aurantiaca*, *Tetrahedron* include malbrancheamide, tajixanthone hydrate and chlorpromazine (CPZ). Another natural inhibitor of CaM is known as melittin (Figure 16), a 26-residue peptide antagonist derived from bee venom. Sung et al. Mol. Cells 27, 167-173 (2009) demonstrated that, in HEK cells, TRPC4 currents were inhibited by W-7 which is a Calmodulin (CaM). However, currents were not inhibited by another CaM antagonist, calmidazolium (CMZ) (Figure 2). This study proves that CaM inhibitors including analogs and derivatives are reasonable candidates for the inhibition of TRPC4 protein function. Such analogs of W-7 can be found in WO-A-2004/071413 and PLOS ONE, vol. 2 (2007), page e545.

Nonspecific and specific modulators of other TRP channel proteins exist. These inhibitors consist of natural endogenous and exogenous agonists and antagonists. These agonists include fatty acids, natural, semi-synthetic and synthetic compounds. TRP channel antagonists also consist of naturally occurring agents such as Thapsigargin. Known TRP channel antagonists also include compound groups such as ureas, cinnamides, carboxamides, and imidazoles. The potency and selectivity of these inhibitors proves that the same compound groups and analogs of these compounds are reasonable candidates for the inhibition of TRPC4 protein function.

It has been proven that the human TRPC4 protein contains multiple ankyrin domains throughout and within the N-terminus along with a coiled-coil domain. The N-terminus of TRPC4 is very important for subunit assembly and pore formation. Two regions in the N-terminus are essential for channel assembly in TRPC channels and more specifically TRCP4; the third and fourth ankyrin repeats and the region downstream the coiled-coil domain. The second and third ankyrin repeats are represented by F59-S137 in TRPC4. Both of these domains are able to self associate but have not been shown to interact with one another. The last 18 amino acids of the region downstream of the coiled-coil domain are represented in TRPC4 by 287-ARLKLAIKYRQKEFVAP-304, and in TRPC6 by 363-SRLKLAIKYEVKKFVAHP-380. These peptides have been identified to be involved in channel assembly of TRPCs and more specifically TRPC4. There are two domains in the TRPC4 protein that are responsible for oligomerization. The first domain contains the N-term ankyrin repeats and the coiled-coil domain (M1-P304) and the second domain corresponds to the putative pore region and the C-terminal tail (I516-L974). Two models exist in which the TRPC4 channel becomes functional upon subunit assembly. One model is that the third ankyrin repeat initiates a molecular zippering process. In this model each interacting domain would have the ability to tetramerize. In another model, the first interaction domain forms a dimer between two subunits and the second domain is responsible for the formation of a dimer between two other subunits. The N-terminal of both TRPC4 and TRPC5 including at least the first ankyrin repeat are essential for both homo and hetero-subunit assembly. TRPC4 protein homo and heteromeric pore formation is critical for protein function; therefore, agents that block TRPC4 multimeric formation are reasonable candidates for TRPC4 protein inhibitors.

Neuropathic pain is a chronic disease resulting from a dysfunction in the nervous system. This nervous system dysfunction often occurs due to peripheral nerve injury concentrated at the dorsal root ganglia (DRG), sensory neurons. Abnormal nervous function arises from injured axons, and from intact nociceptors that share receptivity with the injured nerve. The pathological conditions include prolonged hyperalgesia, allodynia, and loss of sensory function. The classical presentation of neuropathic pain includes ubiquitous pain not otherwise explainable, sensory defects, burning pain, pain to light on the skin and sudden pain attacks without a clear provocation. Inflammation and trauma are major causes of nerve injuries. The genetic disorders causing distorted connectivity, structure or survival of neurons may also result in neuropathic pain.

In order to validate *TRPC4* as a target for therapeutic intervention for neuropathic pain a knockout rat model was created. Gene modification is a process whereby a specific gene, or a fragment of that gene, is altered. This alteration of the targeted gene may result in a change in the level of RNA and/or protein that is encoded by that gene, or the alteration may result in the targeted gene encoding a different RNA or protein than the untargeted gene. The modified gene may be studied in the context of a cell, or, more preferably, in the context of a genetically modified animal.

Genetically modified animals are among the most useful research tools in the biological sciences. An example of a genetically modified animal is a transgenic animal, which has a heterologous (i.e., foreign) gene, or gene fragment, incorporated into their genome that is passed on to their offspring. Although there are several methods of producing genetically modified animals, the most widely used is microinjection of DNA into single cell embryos. These embryos are then transferred into pseudopregnant recipient foster mothers. The offspring are then screened for the presence of the new gene, or gene fragment. Potential applications for genetically modified animals include discovering the genetic basis of human and animal diseases, generating disease resistance in humans and animals, gene therapy, toxicology studies, drug testing, pharmacokinetics and production of improved agricultural livestock.

Identification of novel genes and characterization of their function using mutagenesis has also been shown to be productive in identifying new drugs and drug targets. Creating *in vitro* cellular models that exhibit phenotypes that are clinically relevant provides a valuable substrate for drug target identification and screening for compounds that modulate not only the phenotype but also the target(s) that controls the phenotype. Modulation of such a target can provide information that validates the target as important for therapeutic intervention in a clinical disorder when such modulation of the target serves to modulate a clinically relevant phenotype.

### Brief Summary of the Invention

The invention is defined according to the appended claims. The disclosure relates to methods including compounds, derivatives, antibodies, interfering RNA, biologics, polypeptides, dominant negative effectors, and their use in the treatment of pain by inhibition of TRP channels. In one aspect, the pain inhibition is selected from the group consisting of acute, chronic, neuropathic, nociceptive, visceral, central and peripheral pain signaling.

In another aspect, this disclosure relates to inhibitors, antagonists, and agonists of TRPC4. TRPC4 therapeutic agents and modulators include but are not limited to small molecule inhibitors, compounds, amino acid derivatives, polypeptides, RNA interference agents, natural chemicals, ligand derivatives, and ions. TRPC4 therapeutic agents and modulators are developed for the treatment of neuropathic pain, including but not limited to pain sensations such as nociception, hyperalgesia, allodynia, and loss of sensory function.

Additional advantages of the invention will be set forth in part in the description which follows, and in part will be obvious from the description, or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### Brief Description of the Drawing

This invention, as defined in the claims, can be better understood with reference to the following drawings:
Figure 1: Three-dimensional structures of CaM in its different conformations: (A) calcium free; (B) with calcium; (C) with TFP. D. Johnson et al Biochem. J. 211 (1983).
Figure 2: Current is shown on the Y axis. Inhibitors M1-7 and CMZ are unable to inhibit TRPC4 current, but CaM inhibitor W-7 significantly decreases TRPC4 current.
Figure 3: DNA transposon-mediated insertion mutation in *Rattus norvegicus Trpc4* gene.

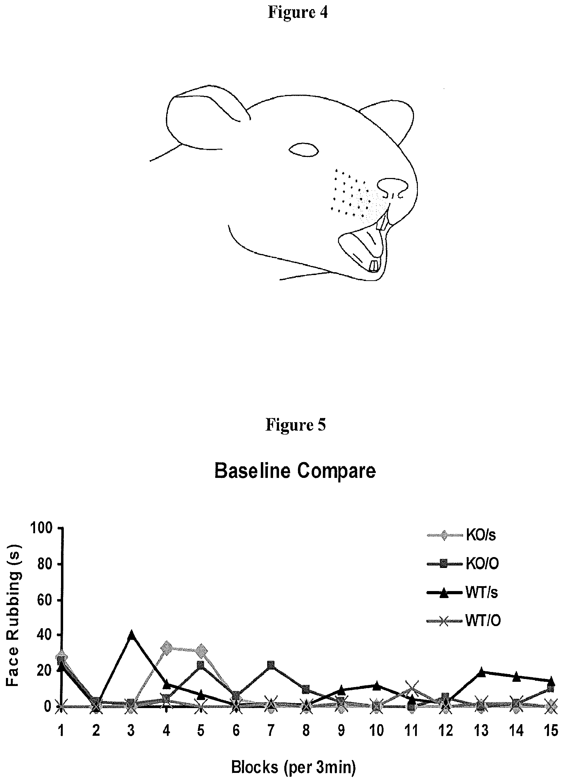

Figure 4: Approximate diffusion area of formalin in the rat upper lip
Figure 5: Baseline face rubbing activity is not different between *Trpc4*+/*-* and wild type F344/N rats.

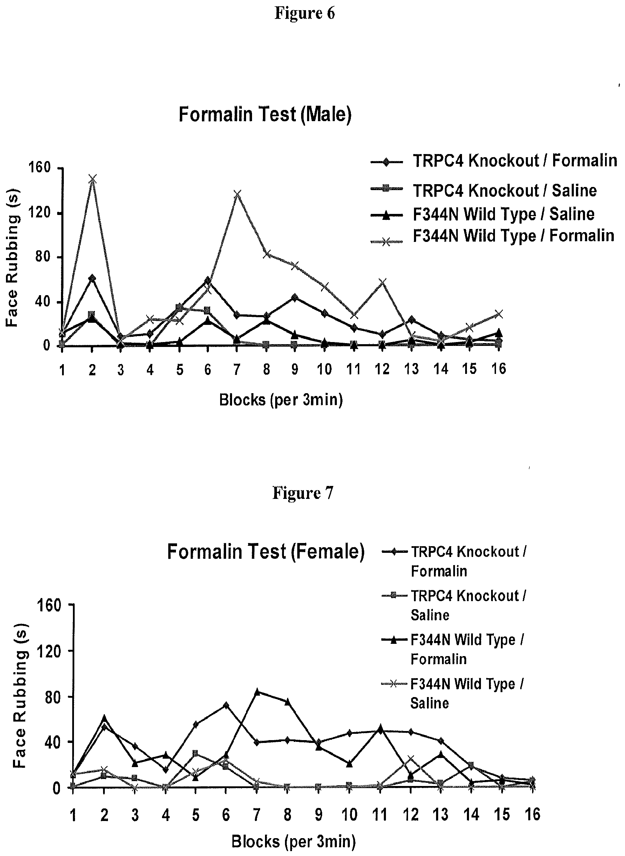

Figure 6: Male formalin test face rubbing activity shows significant difference between *Trpc4*+/*-* and wild type F344/N rats.
Figure 7: Female formalin test face rubbing activity shows no significant difference between *Trpc4*+/*-* and wild type F344/N rats.

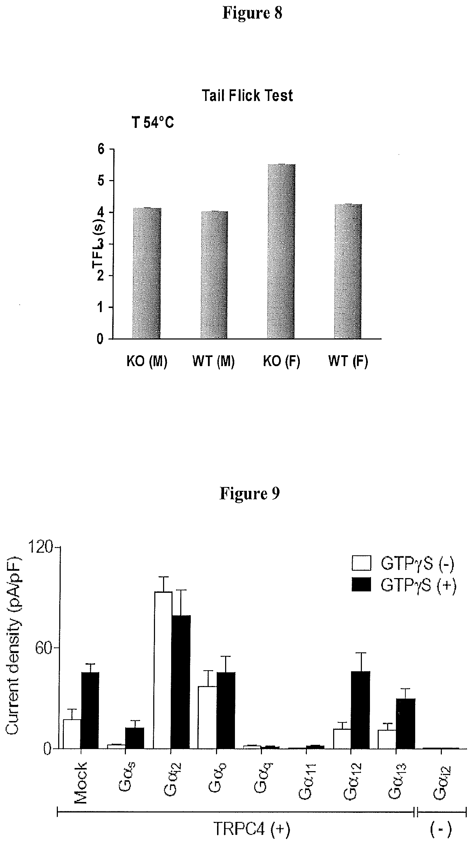

Figure 8: Tail flick test spinal pain threshold testing with the hotplate for male and female *Trpc4*+/*-* and wild type F344/N rats shows no significant change in males and a trend for decreased tail flick latency (s) in the female *Trpc4*+/*-* group.
Figure 9: TRPC4 current density with and without GTPyS stimulation and with constitutive expression of different G proteins. Gai2 clearly activates TRPC4 currents while Gaq/11 clearly inhibits currents. Cells without TRPC4 show no current activation when Gai2 is expressed.

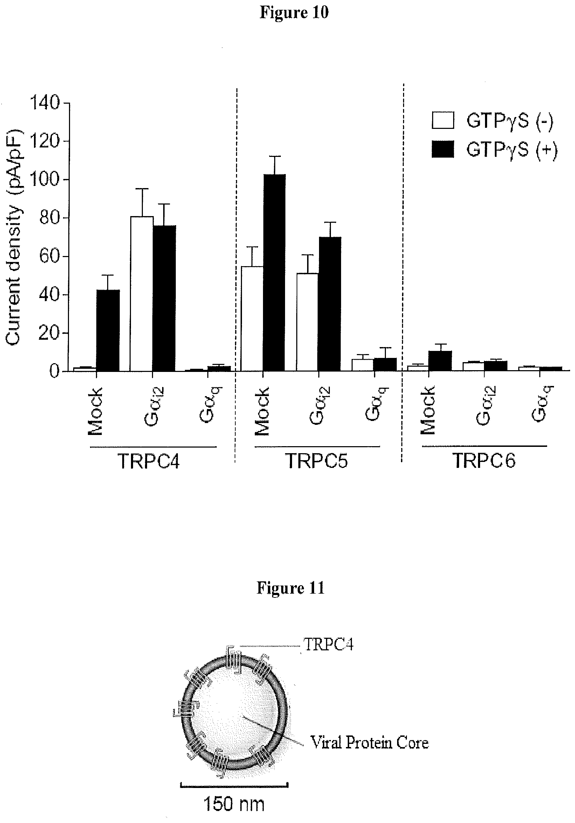

Figure 10: Gai2 is shown to be unable to stimulate currents above the mock in TRPC5 transfected cells. Gaq inhibits both channels
Figure 11: Lipoparticle structure and size. The membrane protein depicted on the lipoparticle is TRPC4.

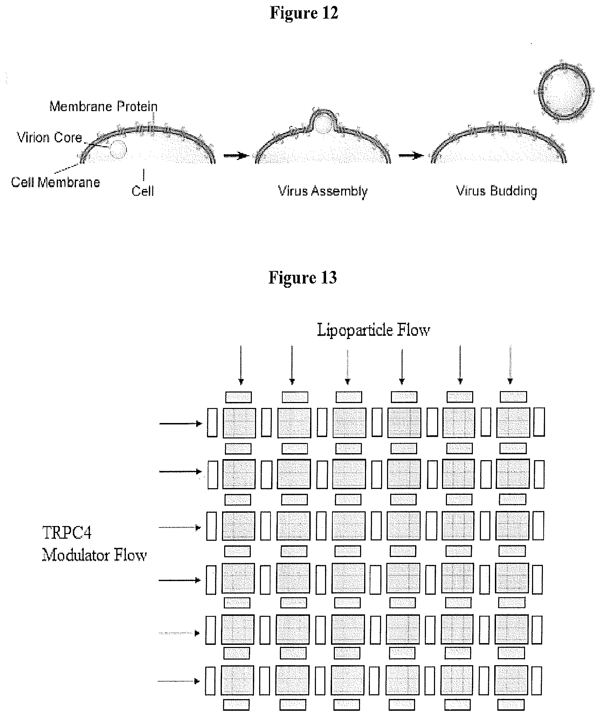

Figure 12: Lipoparticle production process. The cell is co transfected with viral and membrane protein DNA. The membrane protein is overexpressed on the surface of the cell. When the viral component buds our from the cell it pinches off a piece of the cell membrane containing the protein of interest, in this case TRPC4.
Figure 13: Directional flow methods of the lipoparticles containing TRPC4 (vertical) and TRPC4 modulators such as antibodies and compounds (horizontal).

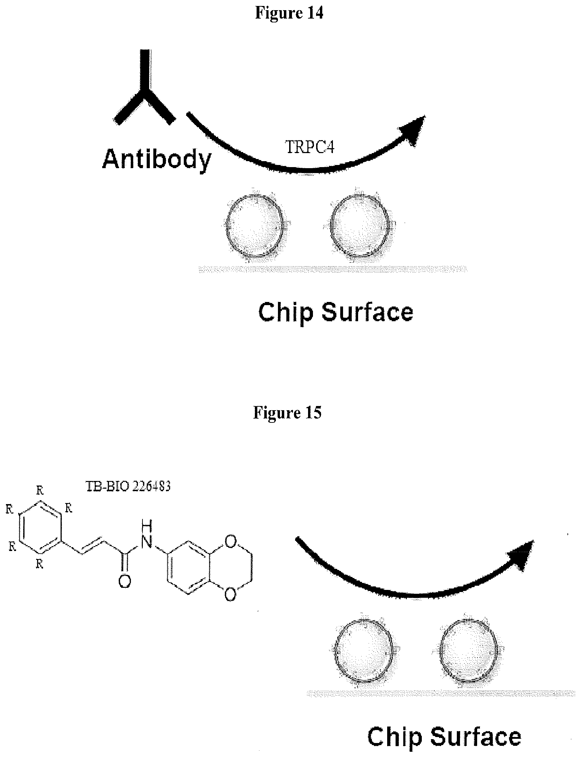

Figure 14: Lipoparticle antibody assay depicted for TRPC4 activity measurement.
Figure 15: Lipoparticle compound assay depicted for TRPC4 activity measurement. The compound depicted is a analog of a proposed Transposagen Biopharmaceuticals TRPC4 modulator, TP-Bio 226483. The "R" represents potential side groups of the compound.
Figure 16: Anticipated critical amino acid mutation profile and activity measurement when in the presence of two proposed Transposagen Biopharmaceutical TRPC4 modulators (TP-Bio 226483 and 6684). The % reactivity on the Y-axis depicts how the critical side groups for TRPC4 modulator binding are discovered.

### Detailed Description of the Invention

### Definitions

The present invention may be understood more readily by reference to the following detailed description of preferred embodiments of the invention and the Examples included therein and to the Figures and their previous and following description. Although any methods and materials similar or equivalent to those described herein can be used in the practice or testing of the present invention, the preferred methods, devices, and materials are now described. All references, publications, patents, patent applications, and commercial materials mentioned herein are referenced for the purpose of describing and disclosing the materials and/or methodologies which are reported in the publications which might be used in connection with the invention. Nothing herein is to be construed as an admission that the invention is not entitled to antedate such disclosure by virtue of prior invention. As used herein, the terms "comprising",
"containing", "having" and "including" are used in their open, non-limiting sense.

Before the present compounds, compositions, articles, devices, and/or methods are disclosed and described, it is to be understood that this invention is not limited to specific synthetic methods, specific recombinant biotechnology methods unless otherwise specified, or to particular reagents unless otherwise specified, as such may, of course, vary. It is also to be understood that the terminology used herein is for the purpose of describing particular embodiments only and is not intended to be limiting.

As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

Ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.

In this specification and in the claims which follow, reference will be made to a number of terms which shall be defined to have the following meanings:
"Optional" or "optionally" means that the subsequently described event or circumstance may or may not occur, and that the description includes instances where said event or circumstance occurs and instances where it does not.

"Pain" is a sensory experience perceived by nerve tissue distinct from sensations of touch, pressure, heat and cold. The range of pain sensations, as well as the variation of perception of pain by individuals, renders a precise definition of pain near impossible. In the context of the present invention, "pain" is used in the broadest possible sense and includes nociceptive pain, such as pain related to tissue damage and inflammation, pain related to noxious stimuli, acute pain, chronic pain, and neuropathic pain.

Pain that is caused by damage to neural structures is often manifest as a neural supersensitivity or hyperalgesia and is termed "neuropathic" pain. Pain can also be "caused" by the stimulation of nociceptive receptors and transmitted over intact neural pathways, such pain is termed "nociceptive" pain.

The level of stimulation at which pain becomes noted is referred to as the "pain threshold." Analgesics are pharmaceutical agents which relieve pain by raising the pain threshold without a loss of consciousness. After administration of an analgesic drug, a stimulus of greater intensity or longer duration is required before pain is experienced. In an individual suffering from hyperalgesia an analgesic drug may have an anti-hyperalgesic effect. In contrast to analgesics, agents such as local anaesthetics block transmission in peripheral nerve fibers thereby blocking awareness of pain. General anaesthetics, on the other hand, reduce the awareness of pain by producing a loss of consciousness.

"Acute pain" is often short-lived with a specific cause and purpose; generally produces no persistent psychological reactions. Acute pain can occur during soft tissue injury, and with infection and inflammation. It can be modulated and removed by treating its cause and through combined strategies using analgesics to treat the pain and antibiotics to treat the infection.

"Chronic pain" is distinctly different from and more complex than acute pain. Chronic pain has no time limit, often has no apparent cause and serves no apparent biological purpose. Chronic pain can trigger multiple psychological problems that confound both patient and health care provider, leading to feelings of helplessness and hopelessness. The most common causes of chronic pain include low-back pain, headache, recurrent facial pain, pain associated with cancer and arthritis pain.

In one aspect, the methods of the disclosure are used to treat "neuropathic pain." Neuropathic pain typically is long-lasting or chronic and can develop days or months following an initial acute tissue injury. Symptoms of neuropathic pain can involve persistent, spontaneous pain, as well as allodynia, which is a painful response to a stimulus that normally is not painful, hyperalgesia, an accentuated response to a painful stimulus that usually a mild discomfort, such as a pin prick, or hyperpathia, a short discomfort becomes a prolonged severe pain. Neuropathic pain generally is resistant to opioid therapy. Neuropathic pain can be distinguished from nociceptive pain or "normal pain," which is pain caused by the normal processing of stimuli resulting from acute tissue injury. In contrast to neuropathic pain, nociceptive pain usually is limited in duration to the period of tissue repair and usually can be alleviated by available opioid and non-opioid analgesics.

By "treating, reducing, or preventing pain" is meant preventing, reducing, or eliminating the sensation of pain in a subject before, during, or after it has occurred. As compared with an equivalent untreated control, such reduction or degree of prevention is at least 5%, 10%, 20%, 40%, 50%, 60%, 80%, 90%, 95%, or 100% as measured by any standard technique known in the art. To treat pain, according to the methods of this invention, the treatment does not necessarily provide therapy for the underlying pathology that is causing the painful sensation. Treatment of pain can be purely symptomatic.

### Overview

While specific configurations and methods describing the present invention are discussed, it should be understood that this is done for illustrative purposes only.

It will be apparent to a person skilled in the pertinent art that this invention can also be employed in a variety of other applications.

### Cells

Cells for use in the method of the disclosure contain functional ion channels. The ion channels of the disclosure are TRP ("the ion channels"). The practitioner may use cells in which the ion channels are endogenous or may introduce either/both of the ion channels into a cell. If ion channels are endogenous to the cell, but the level of expression is not optimum, the practitioner may increase the level of expression of the ion channels in the cell. Where a given cell does not produce the ion channels at all, or at sufficient levels, a nucleic acid encoding the ion channels may be introduced into a host cell for expression and insertion into the cell membrane. The introduction, which may be generally referred to without limitation as "transformation," may employ any available technique. For eukaryotic cells, suitable techniques may include calcium phosphate transfection, DEAE-Dextran, electroporation, liposome-mediated transfection and transduction using retrovirus or other virus, e.g. vaccinia or, for insect cells, baculovirus. General aspects of mammalian cell host system transformations have been described in U.S. Pat. No. 4,399,216. For various techniques for transforming mammalian cells, see Keown et al., Meth. Enzym., 185: 527-537 (1990) and Mansour et al., Nature 336: 348-352 (1988).

It is recognized in the art that there can be significant heterogeneity in a gene sequence depending on the source of the isolated sequence. The invention contemplates the use of conservatively modified variants of the ion channels. Conservatively modified variants applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence to essentially identical sequences. Because of the degeneracy of the genetic code, a large number of functionally identical nucleic acids encode any given protein.

For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein, which encodes a polypeptide, also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid, which encodes a polypeptide, is implicit in each described sequence.

Conservative substitution tables providing functionally similar amino acids are well known in the art. For example, one exemplary guideline to select conservative substitutions includes (original residue followed by exemplary substitution): ala/gly or ser; arg/lys; asn/gln or his; asp/glu; cys/ser; gln/asn; gly/asp; gly/ala or pro; his/asn or gln; ile/leu or val; leu/ile or val; lys/arg or gln or glu; met/leu or tyr or ile; phe/met or leu or tyr; ser/thr; thr/ser; trp/tyr; tyr/trp or phe; val/ile or leu. An alternative exemplary guideline uses the following six groups, each containing amino acids that are conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (I); 5) Isoleucine (I), Leucine (L), Methionine (M), Valine (V); and 6) Phenylalanine (F), Tyrosine (Y), Tryptophan (W); (see also, e.g., Creighton, Proteins, W. H. Freeman and Company (1984); Schultz and Schimer, Principles of Protein Structure, Springer-Verlag (1979)). One of skill in the art will appreciate that the above-identified substitutions are not the only possible conservative substitutions. For example, for some purposes, one may regard all charged amino acids as conservative substitutions for each other whether they are positive or negative. In addition, individual substitutions, deletions or additions that alter, add or delete a single amino acid or a small percentage of amino acids in an encoded sequence can also be considered "conservatively modified variations."

The variant ion channel proteins of the disclosure comprise nonconservative modifications (e.g. substitutions). By "nonconservative" modification herein is meant a modification in which the wildtype residue and the mutant residue differ significantly in one or more physical properties, including hydrophobicity, charge, size, and shape. For example, modifications from a polar residue to a nonpolar residue or vice-versa, modifications from positively charged residues to negatively charged residues or vice versa, and modifications from large residues to small residues or vice versa are nonconservative modifications. For example, substitutions may be made which more significantly affect: the structure of the polypeptide backbone in the area of the alteration, for example the alpha-helical or beta-sheet structure; the charge or hydrophobicity of the molecule at the target site; or the bulk of the side chain. The substitutions which in general are expected to produce the greatest changes in the polypeptide's properties are those in which (a) a hydrophilic residue, e.g. seryl or threonyl, is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g. lysyl, arginyl, or histidyl, is substituted for (or by) an electronegative residue, e.g. glutamyl or aspartyl; or (d) a residue having a bulky side chain, e.g. phenylalanine, is substituted for (or by) one not having a side chain, e.g. glycine. In one aspect, the variant ion channel proteins of the present disclosure have at least one nonconservative modification.

The variant proteins may be generated, for example, by using a PDA system previously described in U.S. Pat. Nos. 6,188,965; 6,296,312; 6,403,312; alanine scanning (see U.S. Pat. No. 5,506,107), gene shuffling (WO 01/25277), site saturation mutagenesis, mean field, sequence homology, polymerase chain reaction (PCR) or other methods known to those of skill in the art that guide the selection of point or deletion mutation sites and types.

The cells used in methods of the present disclosure may be present in, or extracted from, organisms, may be cells or cell lines transiently or permanently transfected or transformed with the appropriate ion channels or nucleic acids encoding them, or may be cells or cell lines that express the required ion channels from endogenous (i.e. not artificially introduced) genes.

### Regulation of gene expression

Expression of the ion channel proteins refers to the translation of the ion channel polypeptides from an ion channel gene sequence either from an endogenous gene or from nucleic acid molecules introduced into a cell. The term "in situ" where used herein includes all these possibilities. Thus in situ methods may be performed in a suitably responsive cell line which expresses the ion channels. The cell line may be in tissue culture or may be, for example, a cell line xenograft in a non-human animal subject.

As used herein, the term "cell membrane" refers to a lipid bilayer surrounding a biological compartment, and encompasses an entire cell comprising such a membrane, or a portion of a cell.

For stable transfection of mammalian cells, depending upon the expression vector and transfection technique used, only a small fraction of cells may integrate the foreign DNA into their genome. In order to identify and select these integrants, a gene that encodes a selectable marker (e.g., resistance to antibiotics) is generally introduced into the host cell along with the gene of interest. Preferred selectable markers include those which confer resistance to drugs, such as G418, hygromycin and methotrexate. A nucleic acid encoding a selectable marker can be introduced into a host cell in the same vector as that encoding the ion channel proteins, or can be introduced in a separate vector. Cells stably transfected with the introduced nucleic acid can be identified by drug selection (e.g., cells that have incorporated the selectable marker gene will survive, while the other cells die).

It should be noted that expression of the ion channel proteins can also be controlled by any of a number of inducible promoters known in the art, such as a tetracycline responsive element, TRE. For example, the ion channel proteins can be selectively presented on the cell membrane by controlled expression using the Tet-on and Tet-off expression systems provided by Clontech (Gossen, M. and Bujard, H. Proc. Natl. Acad. Sci. USA 89: 5547-5551 (1992)). In the Tet-on system, gene expression is activated by the addition of a tetracycline derivative doxycycline (Dox), whereas in the Tet-off system, gene expression is turned on by the withdrawal of tetracyline (Tc) or Dox. Any other inducible mammalian gene expression system may also be used. Examples include systems using heat shock factors, steroid hormones, heavy metal ions, phorbol ester and interferons to conditionally expressing genes in mammalian cells.

The cell lines used in assays of the disclosure may be used to achieve transient expression of the ion channel proteins, or may be stably transfected with constructs that express an ion channel protein. Means to generate stably transformed cell lines are well known in the art. Examples of cells include, but are not limited to Chinese Hamster Ovary (CHO) cells, COS-7, HeLa, HEK 293, PC-12, and BAF.

The level of ion channel expression in a cell may be increased by introducing an ion channel nucleic acid into the cells or by causing or allowing expression from a heterologous nucleic acid encoding an ion channel. A cell may be used that endogenously expresses an ion channel without the introduction of heterologous genes. Such a cell may endogenously express sufficient levels of an ion channel for use in the methods of the invention, or may express only low levels of an ion channel which require supplementation as described herein.

The level of ion channel expression in a cell may also be increased by increasing the levels of expression of the endogenous gene. Endogenous gene activation techniques are known in the art. The level of ion channel expression in a cell may be determined by techniques known in the art, including but not limited to, nucleic acid hybridization, polymerase chain reaction, RNase protection, dot blotting, immunocytochemistry and Western blotting. Alternatively, ion channel expression can be measured using a reporter gene system. Such systems, which include for example red or green fluorescent protein (see, e.g. Mistili and Spector, Nature Biotechnology 15: 961-964 (1997), allow visualization of the reporter gene using standard techniques known to those of skill in the art, for example, fluorescence microscopy. Furthermore, the ability of *Trpc4* to be activated by known positive modulating compounds, such as thrombin, may be determined following manipulation of the ion channel expressing cells.

Cells described herein may be cultured in any conventional nutrient media. The culture conditions, such as media, temperature, pH and the like, can be selected by the skilled artisan without undue experimentation. In general, principles, protocols, and practical techniques for maximizing the productivity of cell cultures can be found in "Mammalian Cell Biotechnology: a Practical Approach", M. Butler, ed. JRL Press, (1991) and Sambrook et al, supra.

The cells can be grown in solution or on a solid support. The cells can be adherent or non-adherent. Solid supports include glass or plastic culture dishes, and plates having one compartment, or multiple compartments, e.g., multi-well plates. The multi-well vessels of the disclosure may contain up to and a number equaling 96 wells. In another aspect, the multi-well vessel comprises greater than 96 wells. In another aspect, the multi-well vessel comprises 384 wells. In yet another aspect, the multi-well vessel comprises 1536 wells.

The number of cells seeded into each well are preferably chosen so that the cells are at or near confluence, but not overgrown, when the assays are conducted, so that the signal-to-background ratio of the signal is increased.

In one aspect of the present disclosure, inhibitors of gene expression of one ion channel are used to reduce gene expression of the other channel. "Reduce gene expression" as used herein refers to reduction in the level of MRNA, protein, or both MRNA and protein, encoded by a gene or nucleotide sequence of interest. Reduction of gene expression may arise as a result of the lack of production of full length RNA.

In one aspect, an inhibitor is a nucleic acid, for example, an anti-sense nucleotide sequence, an RNA molecule, or an aptamer sequence. An anti-sense nucleotide sequence can bind to a nucleotide sequence within a cell and modulate the level of expression of a persistent sodium channel gene, or modulate expression of another gene that controls the expression or activity of a persistent sodium channel. Similarly, an RNA molecule, such as a catalytic ribozyme, can bind to and alter the expression of a persistent sodium channel gene, or other gene that controls the expression or activity of a persistent sodium channel. An aptamer is a nucleic acid sequence that has a three dimensional structure capable of binding to a molecular target, see, e.g., Jayasena, S. D. Clin. Chem. 45: 1628-1650 (1999).

In addition, a selective antagonist can also be a double-stranded RNA molecule for use in RNA interference methods. RNA interference (RNAI) is a process of sequence-specific gene silencing by post-transcriptional RNA degradation, which is initiated by double-stranded RNA (dsRNA) homologous in sequence to the silenced gene. A suitable double-stranded RNA (dsRNA) for RNAI contains sense and antisense strands of, for example, about 21 contiguous nucleotides corresponding to the gene to be targeted that form 19 RNA base pairs, leaving overhangs of two nucleotides at each 3' end (Elbashir, S. M. et al., Nature 411: 494-498 (2001); Bass, B. L. Nature 411: 428-429 (2001); Zamore, P. D. Nat. Struct. Biol. 8: 746-750 (2001). dsRNAs of about 25-30 nucleotides have also been used successfully for RNAi (Karabinos, A. et al., Proc. Natl. Acad. Sci. USA 98: 7863-7868 (2001). dsRNA can be synthesized in vitro and introduced into a cell by methods known in the art.

Antibodies can also be used as an antagonist of ion channel expression. As used herein, the term "antibody" is meant to include polyclonal antibodies, monoclonal antibodies (mAbs), chimeric antibodies, anti-idiotypic (anti-Id) antibodies to antibodies that can be labeled in soluble or bound form, as well as fragments thereof provided by any known technique, such as, but not limited to enzymatic cleavage, peptide synthesis or recombinant techniques.

Polyclonal antibodies are heterogeneous populations of antibody molecules derived from the sera of animals immunized with an antigen. A monoclonal antibody (mAb) contains a substantially homogeneous population of antibodies specific to antigens, which populations contain substantially similar epitope binding sites. MAbs may be obtained by methods known to those skilled in the art. See, for example Kohler, G. et al., Nature 256: 495-497 (1975); U.S. Pat. No. 4,376,110. Such antibodies may be of any immunoglobulin class including IgG, IgM, IgE, IgA, GILD and any subclass thereof. A hybridoma producing a mAb of the present disclosure may be cultivated in vitro, in situ or in vivo. Production of high titers of mAbs in vivo or in situ makes this the presently preferred method of production.

### Ion Channel Activation

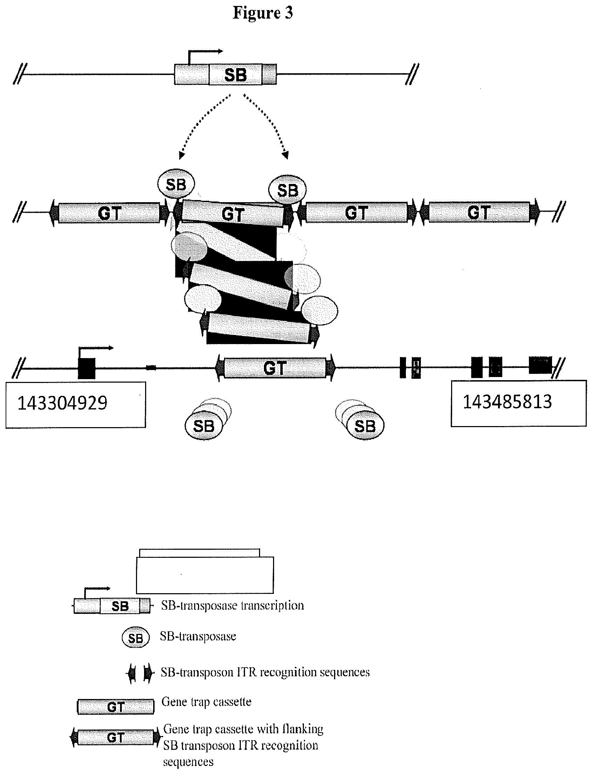

In order to observe ion channel activity, and evaluate whether a test compound can modulate activation, cells expressing the ion channels must be exposed to an activator. There are many methods to activate intracellular calcium stores and many calcium activating agents are known in the art and include, but are not limited to thrombin, adenosine triphosphate (ATP), carbachol, and calcium ionophores (e.g. A23187). While nanomolar increases in calcium concentration ranges are required for *Trpc4* channel activation, the concentration ranges useful for the disclosure are known in the art, e.g., between 10⁻¹⁰ to 10⁻⁴ M for ATP. However, the precise concentration may vary depending on a variety of factors including cell type and time of incubation. The increased calcium concentration can be confirmed using calcium sensitive dyes, e.g., Fluo 3, Fluo 4, or FLIPR calcium 3 dye and single cell imaging techniques in conjunction with Fura2. Changes in membrane potential can also be controlled using cells that cannot generate a *Trpc4* response such as *Trpc4-*CHO cells (see FIG. 3).

Test cells can also be incubated with lower doses of the calcium activating agents described above, such that a fluorescent response that is lower than the maximum achievable response is generated. Generally, the dose is referred to as the effect concentration or EC₂₀₋₃₀, which relates to the effect condition where the fluorescent intensity is 20-30% of the maximal response. As used herein, "EC" refers to effect condition, such that EC₂₀ refers to the effect condition where the fluorescent intensity is 20% of the maximal response is generated. Upon the addition of a second ion channel-specific activating compound, this low response will be increased to at, or near, maximal levels of activation.

In general, agonists and antagonists are used to modulate the ion channels. Agonists" are molecules or compounds that stimulate one or more of the biological properties of a polypeptide of the present disclosure. These may include, but are not limited to, small organic and inorganic molecules, peptides, peptide mimetics and agonist antibodies. The term "antagonist" is used in the broadest sense and refers to any molecule or compound that blocks, inhibits or neutralizes, either partially or fully, a biological activity mediated by a receptor of the present disclosure by preventing the binding of an agonist. Antagonists may include, but are not limited to, small organic and inorganic molecules, peptides, peptide mimetics and neutralizing antibodies.

### Detection of Ion Channel Activation

Movement of physiologically relevant substrates through ion channels can be traced by a variety of physical, optical, or chemical techniques (Stein, W. D., Transport and Diffusion Across Cell Membranes, 1986, Academic Press, Orlando, Fla.). Assays for modulators of ion channels include electrophysiological assays, cell-by-cell assays using microelectrodes (Wu, C. -F. et al., Neurosci 3(9): 1888-99 (1983)), i.e., intracellular and patch clamp techniques (Neher, E. and Sakmann, B., Sci. Amer. 266: 44-51 (1992)), and radioactive tracer ion techniques. Preferably, the effect of the candidate compound is determined by measuring the change in the cell membrane potential after the cell is exposed to the compound. This may be done, for example, using a fluorescent dye that emits fluorescence in response to changes in cell membrane potential and an optical reader to detect this fluorescence.

Voltage sensitive dyes that may be used in the assays and methods of the disclosure have been used to address cellular membrane potentials (Zochowski et al., Biol. Bull. 198: 1-21 (2000)). Membrane potential dyes or voltage-sensitive dyes refer to molecules or combinations of molecules that enter depolarized cells, bind to intracellular proteins or membranes and exhibit enhanced fluorescence. These dyes can be used to detect changes in the activity of an ion channel such as *Trpc4*, expressed in a cell. Voltage-sensitive dyes include, but are not limited to, modified bisoxonol dyes, sodium dyes, potassium dyes and thorium dyes. The dyes enter cells and bind to intracellular proteins or membranes, therein exhibiting enhanced fluorescence and red spectral shifts (Epps et al., Chem. Phys. Lipids 69: 137-150 (1994)). Increased depolarization results in more influx of the anionic dye and thus an increase in fluorescence.

In one aspect, the membrane potential dyes are FMP dyes available from Molecular Devices (Catalog Nos. R8034, R8123). In other aspects, suitable dyes could include dual wavelength FRET-based dyes such as DiSBAC2, DiSBAC3, and CC-2-DMPE (Invitrogen Cat. No. K1016). [Chemical Name Pacific Blue 1,2-ditetradecanoyl-sn-glycero-3-phosphoethanolamine, triethylammonium salt].

Calcium-sensitive fluorescent agents are also useful to detect changes in *Trpc4* activity. Suitable types of calcium-sensitive fluorescent agents include Fluo3, Fluo4, Fluo5, Calcium Green, Calcium Orange, Calcium Yellow, Fura-2, Fura-4, Fura-5, Fura-6, Fura-FF, Fura Red, indo-1, indo-5, BTC (Molecular Probes, Eugene, Oreg.), and FLIPR Calcium3 wash-free dye (Molecular Devices, Sunnyvale Calif.). In one aspect, the intracellular calcium dye is the FLIPR Calcium 3 dye available from Molecular Devices (Part Number: R8091). Additional calcium-sensitive fluorescent agents known to the skilled artisan are also suitable for use in the claimed assay. The calcium-sensitive fluorescent agents can be hydrophilic or hydrophobic.

The voltage- or ion-sensitive fluorescent dyes are loaded into the cytoplasm by contacting the cells with a solution comprising a membrane-permeable derivative of the dye. However, the loading process may be facilitated where a more hydrophobic form of the dye is used. Thus, voltage- and ion-sensitive fluorescent dyes are known and available as hydrophobic acetoxymethyl esters, which are able to permeate cell membranes more readily than the unmodified dyes. As the acetoxymethyl ester form of the dye enters the cell, the ester group is removed by cytosolic esterases, thereby trapping the dye in the cytosol.

The ion channel-expressing cells of the assay are generally preloaded with the fluorescent dyes for 30-240 minutes prior to addition of candidate compounds. Preloading refers to the addition of the fluorescent dye for a period prior to candidate compound addition during which the dye enters the cell and binds to intracellular lipophilic moieties. Cells are typically treated with 1 to 10 µM buffered solutions of the dye for 20 to 60 minutes at 37°C In some cases it is necessary to remove the dye solutions from the cells and add fresh assay buffer before proceeding with the assay.

Another method for testing ion channel activity is to measure changes in cell membrane potential using the patch-clamp technique. (Hamill et al., Nature 294: 462-4 (1981)). In this technique, a cell is attached to an electrode containing a micropipette tip which directly measures the electrical conditions of the cell. This allows detailed biophysical characterization of changes in membrane potential in response to various stimuli. Thus, the patch-clamp technique can be used as a screening tool to identify compounds that modulate activity of ion channels.

Radiotracer ions have been used for biochemical and pharmacological investigations of channel-controlled ion translocation in cell preparations (Hosford, D. A. et al., Brain Res. 516: 192-200 (1990)). As used herein, the phrase "screening for inhibitors of *Trpc4* activity" refers to use of an appropriate assay system to identify novel *Trpc4* modulators from test agents. The assay can be an in vitro or an in vivo assay suitable for identifying whether a test agent can stimulate or suppress one or more of the biological functions of a *Trpc4* molecule or a phospholipase C (PLC) polypeptide. Examples of suitable bioassays include, but are not limited to, assays for examining binding of test agents to a PLC polypeptide or a *Trpc4* polypeptide (e.g., a *Trpc4* fragment containing its ligand binding domain), calcium influx assay, or behavioral analysis. Either an intact PLC or *Trpc4* polypeptide or polynucleotide, fragments, variants, or substantially identical sequences may be used in the screening.

### Assay Detection

Detecting and recording alterations in the spectral characteristics of the dye in response to changes in membrane potential may be performed by any means known to those skilled in the art. As used herein, a "recording" refers to collecting and/or storing data obtained from processed fluorescent signals, such as are obtained in fluorescent imaging analysis.

In some aspects, the assays of the present disclosure are performed on isolated cells using microscopic imaging to detect changes in spectral (i.e., fluorescent) properties. In other aspects, the assay is performed in a multi-well format and spectral characteristics are determined using a microplate reader.

By "well" it is meant generally a bounded area within a container, which may be either discrete (e.g., to provide for an isolated sample) or in communication with one or more other bounded areas (e.g., to provide for fluid communication between one or more samples in a well). For example, cells grown on a substrate are normally contained within a well that may also contain culture medium for living cells. Substrates can comprise any suitable material, such as plastic, glass, and the like. Plastic is conventionally used for maintenance and/or growth of cells in vitro.

A suitable configuration for single cell imaging involves the use of a microscope equipped with a computer system. One example of such a configuration, ATTO's Attofluor RatioVision real-time digital fluorescence analyzer from Carl Zeiss, is a completely integrated work station for the analysis of fluorescent probes in living cells and prepared specimens (ATTO, Rockville, Md.). The system can observe ions either individually or simultaneously in combinations limited only by the optical properties of the probes in use. The standard imaging system is capable of performing multiple dye experiments such as FMP (for sodium) combined with GFP (for transfection) in the same cells over the same period of time. Ratio images and graphical data from multiple dyes are displayed online.

When the assays of the disclosure are performed in a multi-well format, a suitable device for detecting changes in spectral qualities of the dyes used is a multi-well microplate reader. Suitable devices are commercially available, for example, from Molecular Devices (FLEXstation microplate reader and fluid transfer system or FLIPR system), from Hamamatsu (FDSS 6000) and the "VIPR" voltage ion probe reader (Aurora, Bioscience Corp. Calif., USA). The FLIPR-Tetra is a second generation reader that provides real-time kinetic cell-based assays using up to 1536 simultaneous liquid transfer systems. All of these systems can be used with commercially available dyes such as FMP, which excites in the visible wavelength range.

Using the FLIPR system, the change in fluorescent intensity is monitored over time and is graphically displayed as shown, for example in FIG. 1. The addition of ion channel enhancing compounds causes an increase in fluorescence, while ion channel blocking compounds block this increase.

### Candidate Compounds

Candidate compounds employed in the screening methods of this disclosure include for example, without limitation, synthetic organic compounds, chemical compounds, naturally occurring products, polypeptides and peptides, nucleic acids, etc.

Essentially any chemical compound can be used as a potential modulator or ligand in the assays of the disclosure. It will be appreciated that there are many suppliers of chemical compounds, including ChemDiv (San Diego, Calif.), Sigrna-Aldrich (St. Louis, Mo.), Fluka Chemika-Biochemica-Analytika (Buchs Switzerland) and the like.

"Modulating" as used herein includes any effect on the functional activity of the ion channels. This includes blocking or inhibiting the activity of the channel in the presence of, or in response to, an appropriate stimulator. Alternatively, modulators may enhance the activity of the channel. "Enhance" as used herein, includes any increase in the functional activity of the ion channels.

In one aspect, the high throughput screening methods involve providing a small organic molecule or peptide library containing a large number of potential ion channel modulators. Such "chemical libraries" are then screened in one or more assays, as described herein, to identify those library members (particular chemical species or subclasses) that display a desired characteristic activity. The compounds thus identified can serve as conventional "lead compounds" or can themselves be used as potential or actual products.

A combinatorial chemical library is a collection of diverse chemical compounds generated by either chemical synthesis or biological synthesis, by combining a number of chemical "building blocks" such as reagents. For example, a linear combinatorial chemical library such as a polypeptide library is formed by combining a set of chemical building blocks (amino acids) in every possible way for a given compound length (i.e., the number of amino acids in a polypeptide compound). Millions of chemical compounds can be synthesized through such combinatorial mixing of chemical building blocks.

Preparation and screening of combinatorial chemical libraries is well known to those of skill in the art. Such combinatorial chemical libraries include, but are not limited to, peptide libraries (see, e.g., U.S. Pat. No. 5,010,175; Furka Int. J. Pept. Prot. Res. 37: 487-493 (1991) and Houghton et al., Nature 354: 84-88 (1991)). Other chemistries for generating chemical diversity libraries can also be used. Such chemistries include, but are not limited to: peptoids (e.g., PCT Publication No. WO 91/19735), encoded peptides (e.g., PCT Publication No. WO 93/20242), random bio-oligomers (e.g., PCT Publication No. WO 92/00091), benzodiazepines (e.g., U.S. Pat. No. 5,288,514), diversomers such as hydantoins, benzodiazepines and dipeptides (Hobbs et al., Proc. Nat. Acad. Sci. USA 90: 6909-6913 (1993)), vinylogous polypeptides (Hagihara et al., J. Amer. Chem. Soc. 114: 6568 (1992)), nonpeptidal peptidomimetics with glucose scaffolding (Hirschmann et al., J. Amer. Chem. Soc. 114: 9217-9218 (1992)), analogous organic syntheses of small compound libraries (Chen et al., J. Amer. Chem. Soc. 116: 2661 (1994)), oligocarbamates (Cho et al., Science 261: 1303 (1993)), and/or peptidyl phosphonates (Campbell et al., J. Org. Chem. 59: 658 (1994)), nucleic acid libraries (see Ausubel, Berger and Sambrook, all supra), peptide nucleic acid libraries (see, e.g., U.S. Pat. No. 5,539,083), antibody libraries (see, e.g., Vaughn et al., Nature Biotechnology, 14: 309-314 (1996) and PCT/US96/10287), carbohydrate libraries (see, e.g., Liang et al., Science, 274: 1520-1522 (1996) and U.S. Pat. No. 5,593,853), small organic molecule libraries (see, e.g., isoprenoids, U.S. Pat. No. 5,569,588; thiazolidinones and metathiazanones, U.S. Pat. No. 5,549,974; pyrrolidines, U.S. Pat. Nos. 5,525,735 and 5,519,134; morpholino compounds, U.S. Pat. No. 5,506,337; benzodiazepines, U.S. Pat. No. 5,288,514, and the like).

Devices for the preparation of combinatorial libraries are commercially available (see, e.g., 357 MPS, 390 MPS, Advanced Chem Tech, Louisville Ky.; Symphony, Rainin, Woburn, Mass.; 433A Applied Biosystems, Foster City, Calif.; 9050 Plus, Millipore, Bedford, Mass.). In addition, numerous combinatorial libraries are themselves commercially available (see, e.g., ComGenex, Princeton, N.J.; Asinex, Moscow, Russia; Tripos, Inc., St. Louis, Mo.; ChemStar, Ltd, Moscow, Russia; 3D Pharmaceuticals, Exton, Pa.; Martek Biosciences, Columbia, Md.; etc.).

Candidate agents, compounds, drugs, and the like encompass numerous chemical classes, though typically they are organic molecules, preferably small organic compounds having a molecular weight of more than 100 and less than about 10,000 daltons, preferably, less than about 2000 to 5000 daltons. Candidate compounds may comprise functional groups necessary for structural interaction with proteins, particularly hydrogen bonding, and typically include at least an amine, carbonyl, hydroxyl or carboxyl group, preferably at least two of the functional chemical groups. The candidate compounds may comprise cyclical carbon or heterocyclic structures, and/or aromatic or polyaromatic structures substituted with one or more of the above functional groups. Candidate compounds are also found among biomolecules including peptides, saccharides, fatty acids, steroids, purines, pyrimidines, derivatives, structural analogs or combinations thereof.

A variety of other reagents may be included in the screening assay according to the present disclosure. Such reagents include, but are not limited to, salts, solvents, neutral proteins, e.g. albumin, detergents, etc., which may be used to facilitate optimal protein-protein binding and/or to reduce non-specific or background interactions. Examples of solvents include, but are not limited to, dimethyl sulfoxide (DMSO), ethanol and acetone, and are generally used at a concentration of less than or equal to 1 % (v/v) of the total assay volume. In addition, reagents that otherwise improve the efficiency of the assay, such as protease inhibitors, antimicrobial agents, etc. may be used. Further, the mixture of components in the method may be added in any order that provides for the requisite binding.

The compounds identified using the disclosed assay are potentially useful as ingredients or flavorants in ingestible compositions, i.e., foods and beverages as wells as orally administered medicinals. Compounds that modulate taste perception can be used alone or in combination as flavorants in foods or beverages. The amount of such compound(s) will be an amount that yields the desired degree of modulated taste perception of which starting concentrations may generally be between 0.1 and 1000 µM.

### Pain Models

The ability of a compound that selectively reduces *Trpc4*-mediated pain can be confirmed using a variety of well-known assays.

Tail Flick Model: The tail-flick test (D'Amour et al., J. Pharmacol. Exp. and Ther. 72: 74-79 (1941)) is a model of acute pain. A gently-restrained rat is placed on a test stage such that a focused light source beams on the dorsal or ventral surface of the rat's tail. A photosensor is present on the test stage located opposite the light source. To begin the test, the rat's tail blocks the light, thus preventing the light reaching the photosensor. Latency measurement begins with the activation of the light source. When a rat moves or flicks its tail, the photosensor detects the light source and stops the measurement. The test measures the period of time (duration) that the rat's tail remains immobile (latent). Rats are tested prior to administration thereto of a compound of interest and then at various times after such administration.

Rat Tail Immersion Model: The rat tail immersion assay is also a model of acute pain. A rat is loosely held in hand while covered with a small folded thin cotton towel with its tail exposed. The tip of the tail is dipped into a, e.g., 52° C. water bath to a depth of two inches. The rat responds by either wiggling of the tail or withdrawal of the tail from the water; either response is scored as the behavioral end-point. Rats are tested for a tail response latency (TRL) score prior to administration thereto of a compound of interest and then retested for TRL at various times after such administration.

Carrageenan-induced Paw Hyperalgesia Model: The carrageenan paw hyperalgesia test is a model of inflammatory pain. A subcutaneous injection of carrageenan is made into the left hindpaws of rats. The rats are treated with a selected agent before, e.g., 30 minutes, the carrageenan injection or after, e.g., two hours after, the carrageenan injection. Paw pressure sensitivity for each animal is tested with an analgesymeter three hours after the carrageenan injection. See, Randall et al., Arch. Int. Pharmacodyn. 111: 409-419 (1957).

The effects of selected agents on carrageenan-induced paw edema can also be examined. This test (see, Vinegar et al., J. Phamacol. Exp. Ther. 166: 96-103 (1969) allows an assessment of the ability of a compound to reverse or prevent the formation of edema evoked by paw carrageenan injection. The paw edema test is carried out using a plethysmometer for paw measurements. After administration of a selected agent, a carrageenan solution is injected subcutaneously into the lateral foot pad on the plantar surface of the left hind paw. At three hours post-carrageenan treatment, the volume of the treated paw (left) and the untreated paw (right) is measured using a plethysmometer.

Formalin Behavioral Response Model: The formalin test is a model of acute, persistent pain. Response to formalin treatment is biphasic (Dubuisson et al., Pain 4: 161-174 (1977)). The Phase I response is indicative of a pure nociceptive response to the irritant. Phase 2, typically beginning 20 to 60 minutes following injection of formalin, is thought to reflect increased sensitization of the spinal cord.

Von Frey Filament Test (Chang model): The effect of compounds on mechanical allodynia can be determined by the von Frey filament test in rats with a tight ligation of the L-5 spinal nerve: a model of painful peripheral neuropathy. The surgical procedure is performed as described by Kim et al., Pain 50: 355-363 (1992). A calibrated series of von Frey filaments are used to assess mechanical allodynia (Chaplan et al., J. Neurosci. Methods 53: 55-63 (1994)). Filaments of increasing stiffness are applied perpendicular to the midplantar surface in the sciatic nerve distribution of the left hindpaw. The filaments are slowly depressed until bending occurred and are then held for 4-6 seconds. Flinching and licking of the paw and paw withdrawal on the ligated side are considered positive responses.

Chronic Constriction Injury: Heat and cold allodynia responses can be evaluated as described below in rats having a chronic constriction injury (CCI). A unilateral mononeuropathy is produced in rats using the chronic constriction injury model described in Bennett et al., Pain 33: 87-107 (1988). CCI is produced in anesthetized rats as follows. The lateral aspect of each rat's hind limb is shaved and scrubbed with Nolvasan. Using aseptic techniques, an incision is made on the lateral aspect of the hind limb at the mid-thigh level. The biceps femoris is bluntly dissected to expose the sciatic nerve. On the right hind limb of each rat, four loosely tied ligatures (for example, Chromic gut 4.0; Ethicon, Johnson and Johnson, Somerville, N.J.) are made around the sciatic nerve approximately 1-2 mm apart. On the left side of each rat, an identical dissection is performed except that the sciatic nerve is not ligated (sham). The muscle is closed with a continuous suture pattern with, e.g., 4-0 Vicryl (Johnson and Johnson, Somerville, N.J.) and the overlying skin is closed with wound clips. The rats are ear-tagged for identification purposes and returned to animal housing.

The Hargreaves Test: The Hargreaves test (Hargreaves et al., Pain 32: 77-88 (1998)) is also a radiant heat model for pain. CCI rats are tested for thermal hyperalgesia at least 10 days post-op. The test apparatus consists of an elevated heated (80-82.degree. F.) glass platform. Eight rats at a time, representing all testing groups, are confined individually in inverted plastic cages on the glass floor of the platform at least 15 minutes before testing. A radiant heat source placed underneath the glass is aimed at the plantar hind paw of each rat. The application of heat is continued until the paw is withdrawn (withdrawal latency) or the time elapsed is 20 seconds. This trial is also applied to the sham operated leg. Two to four trials are conducted on each paw, alternately, with at least 5 minutes interval between trials. The average of these values represents the withdrawal latency.

Cold Allodynia Model: The test apparatus and methods of behavioral testing is described in Gogas et al., Analgesia 3: 111-118 (1997). The apparatus for testing cold allodynia in neuropathic (CCI) rats consists of a Plexiglass chamber with a metal plate 6 cm from the bottom of the chamber. The chamber is filled with ice and water to a depth of 2.5 cm above the metal plate, with the temperature of the bath maintained at 0-4°C throughout the test. Each rat is placed into the chamber individually, a timer started, and the animal's response latency was measured to the nearest tenth of a second. A "response" is defined as a rapid withdrawal of the right ligated hindpaw completely out of the water when the animal is stationary and not pivoting. An exaggerated limp while the animal is walking and turning is not scored as a response. The animals' baseline scores for withdrawal of the ligated leg from the water typically range from 7-13 seconds. The maximum immersion time is 20 seconds with a 20-minute interval between trials.

Using any of these assays and others known in the art, those skilled in the art recognize that ED.sub.50 values and their standard errors of the mean can be determined using accepted numerical methods, see, e.g., Roger E. Kirk, Experimental Design: Procedures for the Behavioral Sciences, (Wadsworth Publishing, 3.sup.rd ed. 1994).

### Pharmaceutical Compositions

As disclosed herein, a selective modulator of *Trpc4* can be administered to a mammal to modulate in vivo processes involving *Trpc4* such as treating pain, mechanosensation and modifying taste. As used herein, the term "treating pain," when used in reference to administering to a mammal an effective amount of a *Trpc4* antagonist, means reducing a symptom of pain, or delaying or preventing onset of a symptom of pain in the mammal. The effectiveness of a *Trpc4* antagonist in treating pain can be determined by observing one or more clinical symptoms or physiological indicators associated with pain, as described above.

The appropriate effective amount to be administered for a particular application of the methods can be determined by those skilled in the art, using the guidance provided herein. For example, an effective amount can be extrapolated from in vitro and in vivo assays as described herein above. One will recognize that the condition of the patient can be monitored throughout the course of therapy and that the effective amount of a *Trpc4* antagonist that is administered can be adjusted accordingly.

The disclosure also can be practiced by administering an effective amount of a *Trpc4* antagonist together with one or more other agents including, but not limited to, one or more analgesic agents. In such "combination" therapy, it is understood that the antagonist can be delivered independently or simultaneously, in the same or different pharmaceutical compositions, and by the same or different routes of administration as the one or more other agents.

A *Trpc4* antagonist or other compound useful in the invention generally is administered in a pharmaceutical acceptable composition. As used herein, the term "pharmaceutically acceptable" refer to any molecular entity or composition that does not produce an adverse, allergic or other untoward or unwanted reaction when administered to a human or other mammal. As used herein, the term "pharmaceutically acceptable composition" refers to a therapeutically effective concentration of an active ingredient. A pharmaceutical composition may be administered to a patient alone, or in combination with other supplementary active ingredients, agents, drugs or hormones. The pharmaceutical compositions may be manufactured using any of a variety of processes, including, without limitation, conventional mixing, dissolving, granulating, dragee-making, levigating, emulsifying, encapsulating, entrapping, and lyophilizing. The pharmaceutical composition can take any of a variety of forms including, without limitation, a sterile solution, suspension, emulsion, lyophilizate, tablet, pill, pellet, capsule, powder, syrup, elixir or any other dosage form suitable for administration.

It is also envisioned that a pharmaceutical composition can optionally include a pharmaceutically acceptable carriers that facilitate processing of an active ingredient into pharmaceutically acceptable compositions. As used herein, the term "pharmacologically acceptable carrier" refers to any carrier that has substantially no long term or permanent detrimental effect when administered and encompasses terms such as "pharmacologically acceptable vehicle, stabilizer, diluent, auxiliary or excipient." Such a carrier generally is mixed with an active compound, or permitted to dilute or enclose the active compound and can be a solid, semi-solid, or liquid agent. It is understood that the active ingredients can be soluble or can be delivered as a suspension in the desired carrier or diluent. Any of a variety of pharmaceutically acceptable carriers can be used including, without limitation, aqueous media such as, e.g., distilled, deionized water, saline; solvents; dispersion media; coatings; antibacterial and antifungal agents; isotonic and absorption delaying agents; or any other inactive ingredient. Selection of a pharmacologically acceptable carrier can depend on the mode of administration. Except insofar as any pharmacologically acceptable carrier is incompatible with the active ingredient, its use in pharmaceutically acceptable compositions is contemplated. Non-limiting examples of specific uses of such pharmaceutical carriers can be found in Pharmaceutical dosage forms and drug delivery systems (Ansel, H. C. et al., eds., Lippincott Williams & Wilkins Publishers, 7.sup.th ed. 1999); Remington: The Science and Practice of Pharmacy (Gennaro, A. R. ed., Lippincott, Williams & Wilkins, 20.sup.th ed. 2000); Goodman & Gilman's The Pharmacological Basis of Therapeutics (Hardman, J. G. et al., eds., McGraw-Hill Professional, 10.sup.th ed. 2001); and Handbook of Pharmaceutical Excipients (Rowe, R. C. et al., APhA Publications, 4.sup.th edition 2003).

It is further envisioned that a pharmaceutical composition disclosed in the present specification can optionally include, without limitation, other pharmaceutically acceptable components, including, without limitation, buffers, preservatives, tonicity adjusters, salts, antioxidants, physiological substances, pharmacological substances, bulking agents, emulsifying agents, wetting agents, sweetening or flavoring agents, and the like. Various buffers and means for adjusting pH can be used to prepare a pharmaceutical composition disclosed in the present specification, provided that the resulting preparation is pharmaceutically acceptable. Such buffers include, without limitation, acetate buffers, citrate buffers, phosphate buffers, neutral buffered saline, phosphate buffered saline and borate buffers. It is understood that acids or bases can be used to adjust the pH of a composition as needed. Pharmaceutically acceptable antioxidants include, without limitation, sodium metabisulfite, sodium thiosulfate, acetylcysteine, butylated hydroxyanisole and butylated hydroxytoluene. Useful preservatives include, without limitation, benzalkonium chloride, chlorobutanol, thimerosal, phenylmercuric acetate, phenylmercuric nitrate and a stabilized oxy-chloro composition. Tonicity adjustors useful in a pharmaceutical composition include, without limitation, salts such as, e.g., sodium chloride, potassium chloride, mannitol or glycerin and other pharmaceutically acceptable tonicity adjustor. The pharmaceutical composition may be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms.

An antagonist useful in a method of the disclosure is administered to a mammal in an effective amount. Such an effective amount generally is the minimum dose necessary to achieve the desired effect, which can be, for example for treating pain, that amount roughly necessary to reduce the discomfort caused by the pain to tolerable levels or to achieve a significant reduction in pain. For example, the term "effective amount" when used with respect to treating pain can be a dose sufficient to reduce pain, for example, by at least 30%, 40%, 50%, 60%, 70%, 80%, 90% or 100%. The subject receiving the *Trpc4* antagonist can be any mammal or other vertebrate in which modulation of *Trpc4*-associated processes is desired, for example, a human, primate, horse, cow, dog, cat or bird.

Various routes of administration can be useful according to a method of the disclosure. Routes of peripheral administration useful in the methods of the disclosure encompass, without limitation, oral administration, topical administration, intravenous or other injection, and implanted minipumps or other extended release devices or formulations. A pharmaceutical composition useful in the invention can be peripherally administered, for example, orally in any acceptable form such as in a tablet, liquid, capsule, powder, or the like; by intravenous, intraperitoneal, intramuscular, subcutaneous or parenteral injection; by transdermal diffusion or electrophoresis; topically in any acceptable form such as in drops, creams, gels or ointments; and by minipump or other implanted extended release device or formulation.

### Methods

*Trpc4* knockout rats were created by transposon insertional mutagenesis. Genetic modification to Rattus norvegicus neuropathic pain gene Transient receptor potential channel 4 (*Trpc4*) was carried out by a DNA transposon insertional mutagenesis method similar to that described in Nature Genet., 25, 35 (2000). The DNA transposon-mediated genetically modified allele was designated Trpc4Tn(sb-T2/Bart3)2.192Mcwi. The mutant strain symbol for the neuropathic pain rat was designated F344-Trpc4Tn(sb-T2/Bart3)2.192Mcwi. The DNA transposon insertion occurred in chromosome 2, within intron 1 of the rat Trpc4 gene. The sequence tag map position was between base pairs: 143344742-143344909. The sequence tag was:

TATGTTTAGGCCATGGAGATAAGAGGCATCTTCCAGAGTTAGG
AATTACATACATCTGCACTTATGTATCACGATTATGCTTCTGAA
TGCACCTAACAAGAGCTCGAGGAGAAACCATGCAGAGAGGAA
CAATTGAAAAGGAAGTACATTGTGCAGACTGCTTCCTAG.

Thus, a DNA transposon was inserted into the Trpc4 gene of Rattus norvegicus and Western blot analysis indicated that the gene was completely inactive. To study the effect of Trpc4 on neuropathic pain two mechanical pain tests, diabetes induced, and drug induced studies were done on the animal models. First the spared nerve injury (SNI) operation was performed on a set of controls and Trpc4 knockout rats. Once the sciatic nerve and terminal branches were exposed on the lateral side of the back paw the peroneal and tibial nerves were ligated. Groups of wild type and Trpc4 knockout rats also underwent partial nerve injury (PNI) by tightening the sciatic nerve of approximately 1/3-1/2 of the normally functional diameter. From the SNI and PNI neuropathic pain induced rats, controls and Trpc4 knockouts were assessed for response to mechanical pain via von Frey filaments as described above. It was observed that under both pain models *Trpc4* knockout rats exhibited 10-20% of the pain response that was displayed in wild type control rats. This mechanical test indicated that *Trpc4* knockout rats were indeed models for neuropathic pain.

Since many patients with diabetes mellitus also suffer from hyperalgesia-derived neuropathic pain, the *Trpc4*(-/-) rats were studied for this indication. Groups of control and *Trpc4* knockout rats were treated with streptozotocin (STZ) in order to induce diabetes. Rats were defined to be in a diabetic state if hyperglycemia and glucosuria occurred. The mechanical induced von Frey filament method was again used to establish altered pain response. When STZ non-treated control rats were compared to STZ diabetic control rats the pain response to mechanical induction was very drastic. The control non-treated rats maintained the threshold as expected. However, the STZ treated diabetic rats threshold for pain was decreased by 40%. Therefore, the STZ treated control rats were 40% more sensitive to mechanical induced pain. When STZ-induced control and *Trpc4-*/*-* rats were compared for mechanical induced sensitivity to pain the change was significant. These data suggest that the *Trpc4* knockout rat is a model for diabetes-associated neuropathic pain.

Cancer patients who are treated with chemotherapy drugs such as paclitaxel often display sensory abnormalities and symptoms of neuropathic pain such as sudden unexplainable pain attacks. In order to study the effects of cation channels on paclitaxel induced pain, *Trpc4-*/*-* rats were treated with the anti-cancer drug. Since one of the major symptoms of patients who are treated with paclitaxel is cold allodynia, control and *Trpc4*-/- rats were exposed to paclitaxel and tested for acetone cold response. Previously control non-paclitaxel treated and paclitaxel treated WT rats were studied for differences in cold allodynia. Based on established cold score calculations the non-treated rats scored an average of 8 while the paclitaxel treated WT rats scored an average of 15. These data indicate that paclitaxel treated rats are indeed hypersensitive to cold induced pain. When control and *Trpc4*-/- treated rats were compared the difference in pain sensitivity was dramatic. *Trpc4* knockout rats which were treated with paclitaxel displayed a cold score of 9. The reversion to nearly wild type cold allodynia score was remarkable in *Trpc4* knockout rats. These data implicate *Trpc4* as a key mediator of drug and chemical (paclitaxel) induced neuropathic pain.

### Trpc4+/- rat models for nociception

*Trpc4*+/- and F344/N wild-type rats were used in two classic studies for nociception; (1) The formalin test applied to the face (2) The hotplate test

### The orofacial formalin test

Each animal is placed in a new clean see-through cage for a 10-min period to acclimatize to the new environment before the formalin injection. 1.5% formalin, 50 µl, is injected, s.c. into the upper lip, just lateral to the nose. a 28_{G}½ (0.36 x 13 mm) sterile needle mounted on a 0.5cc U-100 Insulin Syringe is inserted into the lip close to the border between hairy and glabrous skin and advanced up to the hilt towards the vibrissal pad. Thus, the formalin solution spreads out within the upper lip and the rostral half of the vibrissal pad. Injections directed to the center of the vibrissal pad have also successfully been carried out. The 1.5% formalin (0.55% formaldehyde), must be freshly prepared each day to improve the sensitivity of the model, to allow the detection of both hypo- and hyperalgesic effects, and to minimize suffering of the animals. Controls are injected with an equal volume of saline at the same site and in the same manner. Immediately after the injection, the animals are placed back in their home cage for a 45-min observation period. A video camera linked with a computer recording program for offline data analysis records their behavior without the presence of investigators in the room.

### Results

The number of seconds that the animals spends rubbing the injected perinasal area (whisker pad, upper lip and nostril) with the ipsilateral or both of forepaw or ipsilateral hindpaw, + head flip is defined as the nociceptive activity. The recording time (45 min) is generally divided into 15 blocks of 3 min for a time course analysis.

### Results

Baseline face rubbing activity is not different between *Trpc4*+/- and wild type F344/N rats (data not shown). Rats are pain free in the normal uninjured condition. Rats were monitored for a 45min period.

Formalin 1.5% 50µl, sc. injected onto the one side upper lip. The animals show a nociceptive activity immediately after injection: Rubbing face and flipping the head lasting for 25-30min. These activities are present with two phases, phase I and phase II responses typical of formalin test. The baseline is the first value. There are some interesting differences in two experimental groups: *Trpc4*+/- male rats show both reduced Phase I and Phase II activity compared to WT that have a robust response in both phases. WT male rats have more face rubbing and head flip activity than *Trpc4*+/- rats in the male group, but not in female group. *Trpc4* rats showed a delayed start of the activity of about 45s to 1.2 min compared to 10-12s for the wild type (not evident at this time scale)

### Tail Flick Test

The tail-flick test is widely used as an experimental model of spinal nociception (pain threshold testing). Rats are handled for 3 min and habituate to the testing room for 1 h on two occasions before the day of testing and again on the day of testing. The rats are removed from their home cage and gently restrained in a towel. The tail (the proximal one third) is immersed in 54°C water. This temperature is used to reduce the effects of stress that can contribute to the measure (d'Amore et al. 1992). The latency of the flick of the tail to bring the tail out of the water is recorded three times; each time separated by 10 s, and the average of the three measures is calculated. All rats respond within 5s, before the predetermined cut-off of 10s used to prevent tissue damage the event the rats have not responded. All tail flick testing is performed between 9:00 A.M. and 1:00 P.M. Notes are taken at each trial by the experimenter as to whether the rat makes an audible vocalization after insertion of the tail in the water. No amplification or modification is done.

### Results

The spinal pain threshold testing with the hotplate for male rats shows that there is no difference between *Trpc4*+/- and WT groups. There is a trend for decreased tail flick latency (s) in the female *Trpc4*+/- group (insufficient numbers so far). Vocalization occurs in the *Trpc4*+/- female group during the testing events.

### TRPC4 antagonist screening and development

Armed with the newly gained knowledge from *Trpc4* knockout rats and the fact that there are no known *Trpc4* antagonists, multiple compounds, derivatives, polypeptides, biologics and antibodies were developed in order to specifically inhibit *Trpc4.*

### Patch clamp assay

The patch-clamp technique, also referred to as micropipette-based measurement, is a laboratory technique in electrophysiology that allows the study of single or multiple ion channels in cells. This assay provides direct measurement of ionic flux. Automated patch-clamp techniques using a conventional micropipette have been developed. These systems gather an abundant amount of data by whole-cell voltage clamping. An alternative to automated pipette patch clamping, the planar patch-clamp device utilizes a micro-sized pore on a flat substrate for cell trapping and signal recording. A single cell is sequestered within the pore by either suction or electrokinetic force. This system allows high throughput, parallel and automated measurement of ion channel function.

Cells membranes were isolated and prepared from rat WT, *Trpc4*+/- and *Trpc4-*/*-* tissues. Further manipulation was undergone in order to seclude the TRPC4 ion channel as the only functional ion channel on the membrane of the cells. The individual cells were positioned correctly for either pipette based or planar or pore based patch clamp assays. Experiments were conducted and current traces were recorded from the voltage-clamp experiment. Voltage steps were evoked between 100 and +80 mV in 20 mV intervals for 200 ms, from a holding potential of 80 mV. WT cells displayed significant channel activation when the holding potential was higher than 20 mV. However, *Trpc4*+/- cells significantly lower leak subtracted currents (pA) when voltage was provided. As expected the cells derived from *Trpc4-*/*-* rats only displayed leak baseline current levels when voltage was applied. Human cell membranes of multiple types including HEK and neuronal cells were prepared in the same fashion. Only the human TRPC4 protein was left functional when patch clamp studies were done. When voltage was applied, human cell membranes emitted WT currents, significantly decreased *TRPC4*+/- currents, and only displayed baseline leak currents for *TRPC4-*/*-* cell membranes.

A high throughput automated patch clamp assay was run with 10,000 candidate compounds in order to discovery a *TRPC4* antagonist. When antagonism was recorded to decrease patch clamp currents at or lower than *TRPC4*+/- recordings the compound was selected for further development. In order to discover a TRPC4 specific inhibitor the same patch clamp technique was run with the selected inhibitors and compared to all TRP channels and other membrane ion channels. Candidates that displayed specific and potent TRPC4 protein inhibition were selected as antagonists for animal studies and future clinical trials for neuropathic pain.

### Lipoparticle Based Discovery Methods

Lipoparticles are pseudotypes which are described as containing functional features of a cell and a viral particle. The viral particle or viron is viral envelope deficient and is therefore non-infectious. The viron core is surrounded by a bi-lipid membrane which harbors the membrane protein, TRPC4 (Figure 61). The lipoparticles are produced by co-transfection of viral core and membrane protein DNA into the cell. The membrane protein is over-expressed on the surface of the cell. The viral core assembles within the cell. The viron buds out of the cell and pinches off a piece of the cell membrane which contains one or multiple copies of TRPC4 (Figure 62). This method is very efficient for the screening of potential modulators for pain membrane protein TRPC4, because 1 µL of lipoparticles can contain as many proteins as 10,000 cells expressing 1X10^6 receptor membrane proteins each. Lipoparticle studies are also not subject to manipulation from cytoplasmic contaminants.

### Lipoparticle Antibody Binding Assays

Lipoparticles harboring TRPC4 on the membrane surface are used on chips called bio-sensors which have multiple flow sensors and are extremely high throughput. The lipoparticles are flowed onto the biosensor in the vertical direction. The antibody which will be used for TRPC4 modulation is then flowed onto the biosensor chip in the horizontal direction (Figure 63). The first step of the antibody binding assay is to immobilize the lipoparticle on the chip. This is done in several ways. The first method is to covalently immobilize a lectin carbohydrate binding protein onto the chip. The lectin then binds to lipoparticle surface carbohydrates as they pass, immobilizing it. The second method is to capture the lipoparticle with an antibody for a surface membrane protein. In this method the lipoparticle displays TRPC4 and another membrane protein on its surface. An antibody for the non-TRPC4 protein is covalently immobilized on the chip. The lipoparticles then bind to the antibody and are themselves immobilized while leaving TRPC4 free for assay studies. Different concentrations of antibody plus a control (buffer) are flowed horizontally across the surface of the chip simultaneously. The antibodies targeting the TRPC4 proteins on the surface of the lipoparticle interact (Figure 64). The bio-sensor software will measure the kinetics of the antibody K(on), K(off), K(D) by response resonance measurements. Development of TRPC4 specific, high-affinity monoclonal antibodies is done using the Lipoparticle method.

### Lipoparticle Compound Binding Assays

In the same method, lipoparticles can be immobilized on the surface of the bio-sensor chip by either the carbohydrate binding protein or antibody capture methods described above. The lipoparticles will display TRPC4 on their surface. Compounds which are analogs of TRPC4 inhibitors are then flowed through the chip and a binding profile is calculated be response resonance measurements (Figure 65).

### Lipoparticle shotgun mutagenesis, identification of critical TRPC4 residues

In order to determine critical residues for TRPC4 interaction with proteins, ligands, antibodies, and compounds a complete mutation library is formed. The library is composed of unique TRPC4 amino acid sequences which have a mutation in every residue position on the protein. Each of the unique mutant sequences are arrayed and expressed in 384 well plates. The plates become assays for TRPC4 interaction with other proteins such as other TRPs (TRPC5), ligands such as G-proteins, and with compounds. The mutant screens also serve as functional assays. Ca2+ flux is a good measurement of function for TRPC4.

By measuring antibody and compound specific interactions with TRPC4 critical amino acid residues are able to be identified. For example, TRPC4 glutamate (E) residue 466 in the second extracellular domain may be found to be critical for TRPC4 modulator binding. Once the critical residues are identified for binding with antibodies and compound modulators a mutant library containing all amino acid changes for that single residue is created. The reactivity of each mutant residue is measured when in the presence of proposed TRPC4 modulators. In this way, side groups such as the charges of carbonyl or carboxylate groups in an anticipated critical residue E466 are identified as essential for TRPC4 modulator binding (Figure 66). These critical amino acid interactions are further studied for their kinetics by creating lipoparticles which harbor TRPC4 wild-type, and different mutant variants which have been found to be important in modulator binding. This strategy allows for the study of binding interaction kinetics (K(on), K(off), K(D))of different modulators such as antibodies and compounds with different variants of TRPC4. This is a critical recipe for the development of a portfolio of effective and non-toxic TRPC4 modulators.

### Classes of Trpc4 inhibitors

CaM inhibitors and more specifically membrane CaM inhibitors have the ability to decrease *Trpc4* activity specifically. Therefore, *Trpc4* inhibition for the treatment of pain involves CaM inhibitor and more specifically membrane CaM inhibitors and more specifically naphthalenesulfonamide derivatives, such as, but not limited to W-7, W-12, W-13 (Table 2)

The most studied and well known CaM inhibitors are analogs of trifluoroperazine (TFP). Therefore, *Trpc4* inhibition for the treatment of pain involves the use of all phenothiazine analogs (Table 3).

Arylakylamine derivatives have been shown to interact with CaM. Therefore, *Trpc4* inhibition for the treatment of pain involves the use of Arylalkylamine analogs (Table 4).

Bis-indole derivatives have been shown to interact with CaM. Therefore, *Trpc4* inhibition for the treatment of pain involves the use of Bis-indole analogs (Table 5).

Alkaloid derivatives have been found to inhibit CaM. Therefore, *Trpc4* inhibition for the treatment of pain involves alkaloid analogs (Table 5).

Xanthone derivatives have been found to inhibit CaM. Therefore, *Trpc4* inhibition for the treatment of pain involves xanthone analogs (Table 7).

Natural CaM inhibitors have been found in fungus and other organisms such as bees. One such example is melittin, which is a component of bee venom. Melittin is a peptide consisting of 26 amino acids with the sequence: GIGAVLKVLTTGLPALISWIKRKRQQ. Therefore, *Trpc4* inhibition for the treatment of pain involves natural products such as peptide and compound analogs (Table 8).

Benzylamine derivatives have been found to inhibit CaM. Therefore, *Trpc4* inhibition for the treatment of pain involves benzylamine analogs (Table 9).

Methylxanthine derivatives have been found to inhibit CaM. Therefore, *Trcp4* inhibition for the treatment of pain involves methylxanthine analogs (Table 10).

Sphingosine derivatives have been found to inhibit CaM. Therefore, *Trpc4* inhibition for the treatment of pain involves sphingosine analogs (Table 11).

Benzenesulfonamide derivatives have been found to inhibit CaM. Therefore, *Trpc4* inhibition for the treatment of pain involves benzenesulfonamide analogs (Table 12).

Synthetic analogs of the TRP channel agonist capsaicin have been proven to inhibit or block TRP channels. Therefore, *Trpc4* inhibition for the treatment of pain involves capsaicin analogs (Table 13).

TRP channels, especially TRPC channels and more specifically Trpc4, are inhibited by Diphenylborinic acid derivatives. Therefore, *Trpc4* inhibition for the treatment of pain involves the use of Diphenylborinic acid analogs (Table 14).

Phorbol ester's and more specifically diterpene phorbol ester's have been found to inhibit the function of *Trpc4.* Therefore, *Trpc4* inhibition for the treatment of pain involves the use of phorbol ester's and more specifically diterpene phorbol ester analogs (Table 15).

Cinnamide derivatives have been proven to inhibit TRP channels. Therefore, *Trpc4* inhibition for the treatment of pain involves the use of cinnamide analogs (Table 16).

Daphnetoxin derivatives have been shown to inhibit TRP channels. Therefore, *Trpc4* inhibition for the treatment of pain involves the use of daphnetoxin analogs (Table 17).

Pyrazine derivatives have been shown to exhibit inhibitory activity on TRP channels. Therefore, *Trpc4* inhibition for the treatment of pain involves analogs composed of pyrazine or pyrazine derivatives (Table 18).

Isoquinolin derivatives have been shown to inhibit TRP channels. Therefore, *Trpc4* inhibition for the treatment of pain involves the use of isoquinolin analogs (Table 19).

Cyclohexanecarboxamide derivatives have been shown to inhibit TRP channels. Therefore, *Trpc4* inhibition for the treatment of pain involves cyclohexanecarboxamide analogs (Table 20).

Lactone derivatives, and more specifically sesquiterpene lactone derivatives have been shown to inhibit TRP channels. Therefore, *Trpc4* inhibition for the treatment of pain involves the use of lactone derivatives and more specifically sesquiterpene lactone analogs (Table 21).

Yohimban derivatives have been shown to inhibit TRP channels. Therefore, *Trpc4* inhibition for the treatment of pain involves the uses of yohimban analogs (Table 22).

Cannabidiol derivatives have been shown to inhibit TRP channels. Therefore, *Trpc4* inhibition for the treatment of pain involves the use of cannabidiol analogs (Table 23).

Adenosine nucleotides have been shown to inhibit TRP channels. Therefore, *Trpc4* inhibition for the treatment of pain involves the use of adenosine analogs (Table 24).

Polyamines such as spermine derivatives have been shown to inhibit TRP channels. Therefore, *Trpc4* inhibition for the treatment of pain involves the use of spermine analogs (Table 25).

Camphor derivatives are known to inhibit TRP channels. Therefore, *Trpc4* inhibition for the treatment of pain involves the use of camphor analogs (Table 26).

Ethanol derivatives are known to inhibit TRP channels. Therefore, *Trpc4* inhibition for the treatment of pain involves the use of ethanol analogs (Table 27).

Amiloride derivatives are known to inhibit TRP channels. Therefore, *Trpc4* inhibition for the treatment of pain involves the use of amiloride analogs (Table 28).

Azole and imidazole derivatives are known to inhibit TRP channels. Therefore, *Trpc4* inhibition for the treatment of pain involves the use of azole and imidazole analogs (Table 29).

Verapamil derivatives are known to inhibit TRP channels. Therefore, *Trpc4* inhibition for the treatment of pain involves the use of verapamil analogs (Table 30).

Di(acrylalkyl)- and Aryl(arylakyl) urea derivatives have been known to inhibit TRP channels. Therefore, *Trpc4* inhibition for the treatment of pain involves the use of Di(acrylalkyl)- and Aryl(arylakyl) urea analogs (Table 31).

Diaryl ethers and amine derivatives have been shown to inhibit TRP channels. Therefore, *Trpc4* inhibition for the treatment of pain involves the use of diaryl ethers and amine analogs (Table 32).

Carboxamides derivatives have been shown to inhibit TRP channels. Therefore, *Trpc4* inhibition for the treatment of pain involves the use of carboxamide analogs (Table 33).

Pyridine derivatives have been shown to inhibit TRP channels. Therefore, *Trpc4* inhibition for the treatment of pain involves the use of pyridine analogs (Table 34).

Ammonium derivatives have been known to inhibit TRP channels. Therefore, *Trpc4* inhibition for the treatment of pain involves the use of ammonium analogs (Table 35).

Citral derivatives have been known to inhibit TRP channels. Therefore, *Trpc4* inhibition for the treatment of pain involves the use of citral analogs (Table 36).

Arsenic hydrides such as arsane are known to inhibit TRP channels. Therefore, *Trpc4* inhibition for the treatment of pain involves the use of arsenic hydrides derivatives (Table 37).

Non-selective inhibitors interact not only with the ligand active site but also block aqueous pores of TRP channels. Ruthenium derivatives are important nonselective pore blockers and have been shown to inhibit TRP channels. Other non-selective TRP pore blockers include organic dyes, aminoglycoside antibiotics, venom from the spider *Angelenopsis aperta*, acylpolyamine toxins. Therefore, *Trpc4* inhibition for the treatment of pain involves the use of non-selective TRP pore blockers including ruthenium analogs (Table 38).

### TRP Channel agonist modification to reverse biological activity

The TRP channel family is activated and regulated by a number of exogenous and endogenous chemicals and ligands. The mechanism of inhibition by competition for the active site of agonists has been a proven method for TRP antagonism. Many potent agonists can be modified in a way which alters its biological activity and generates a powerful TRP antagonist as opposed to agonist. Therefore, *Trpc4* inhibition for the treatment of pain involves the use of analogs of all TRP channel agonists such as but not limited to the following.

Biogenic amines are known to be agonists of TRP channels. Therefore, *Trpc4* inhibition for the treatment of pain involves the use of biogenic amine analogs (Table 39).

Oxygenated eicosatetraenoic acids are known to be agonists of TRP channels. Therefore, *Trpc4* inhibition for the treatment of pain involves the use of oxygenated eicosatetraenoic acid derivatives (Table 40).

Capsaicinoid and capsinoid derivatives are known to be agonists of TRP channels. Therefore, *Trpc4* inhibition for the treatment of pain involves the use of capsaicinoid and capsinoid analogs (Table 41).

Phenylpropanoids are known to be agonists of TRP channels. Therefore, *Trpc4* inhibition for the treatment of pain involves the use of phenylpropanoid analogs (Table 42).

Acetoamide derivatives are known to be agonists of TRP channels. Therefore, *Trpc4* inhibition for the treatment of pain involves the use of acetoamide analogs (Table 43).

Sulfur containing amino acid derivatives are known to be agonists of TRP channels. Therefore, *Trpc4* inhibition for the treatment of pain involves the use of sulfur containing amino acid analogs (Table 44).

Phenylborate derivatives are known to be TRP channel agonists. Therefore, *Trpc4* inhibition for the treatment of pain involves the use of phenylborate analogs (Table 45).

Uricosuric derivatives are known to be TRP channel agonists. Therefore, *Trpc4* inhibition for the treatment of pain involves the use of uricosuric analogs (Table 46).

Cannabinoid derivatives are known to by TRP channel agonists. Therefore, *Trpc4* inhibition for the treatment of pain involves the use of cannabinoid analogs (Table 47).

Cembrane diterpenoid derivatives are known to be TRP channel agonists. Therefore, *Trpc4* inhibition for the treatment of pain involves the use of cembrane diterpenoid analogs (Table 48).

### TRP channel modulator side group structural configurations

Each compound which is involved in TRP channel activation (agonist) or inactivation (antagonist) consists of core structures and multiple side group possibilities. The disclosure is composed of each compound group consisting of the structural core and analogs which include side groups designated as "R" groups. The structural core analogs are composed of all permutations and combinations of "R" groups when R includes but is not limited to: alcohols; aldehydes; aldehydes; butyraldehyde; formaldehyde; aldehydes and ketones; aldehydes or ketones with other functional groups; aldehydes, ketones with other functional groups; kepone; chlordecone; aliphatic hydrocarbons; aliphatic nitriles and cyanates; acetonitrile; acrylonitrile; aliphatic nitriles; aliphatic nitrosamines; aliphatic nitrosamines; *n*-nitrosodimethylamine (ndma); aliphatic organophosphorous compounds; aliphatic organophosphorous compounds; dibutyl phosphate; tributyl phosphate; alkanes and cyclic alkanes; alkanes and cyclic alkanes; cyclohexane; decane, *n*; heptanes; hexadecane; hexanes; isobutane; methane; methyl cyclohexane; nonane; octane, iso; isooctane; pentanes; propane; alkenes, cyclic alkenes, and dienes; alkenes, cyclic alkenes, and dienes; ethylene; propylene; alkyl halides; alkynes; aluminum; americium; amides; amides; carbofuran; dimethyl acetamide; amines; antimony; aromatic amines and diamines; aniline; aromatic amines and diamines; atrazine; benzidine; chloroaniline, 4; chloroaniline(bis) methylene; methylene bis(chloroaniline); mboca;; dichlorodiaminodiphenyl methane; dichlorobenzidine, 3,3; propazine; aromatic nitriles and cyanates; aromatic nitriles; toluene diisocyanate; aromatic nitro compounds; aromatic nitro compounds with other functional groups; aromatic nitro compounds with other functional groups; pentachloronitrobenzene; aromatic organophosphorous compounds; aromatic organophosphorous compounds; parathion; aromatics with halogenated side chain; aromatics with halogenated side chain; benzyl chloride; chlorotoluene, alpha; ddt; trichloro(chlorophenyl,4-bis) ethane; arsenic; azo compounds; azo compounds; azobenzene; azo compounds, hydrazine derivatives; barium; benzene and monosubstituted benzene hydrocarbons; benzene; benzene and monosubstituted benzene hydrocarbons; biphenyl; cumene; isopropyl benzene; methylethyl benzene; ethylbenzene; styrene; toluene; benzene and substituted benzene hydrocarbons; benzene, toluene, ethylbenzene, and xylene (btex); beryllium; bismuth; bromide ion; cadmium; calcium; carbon compounds; carbon compounds; carbon monoxide; carboxylic acids; acetic acid; acrylic acid; propenoic acid; benzoic acid; carboxylic acids; carboxylic acids and derivatives; carboxylic acids with other functional groups; carboxylic acids with other functional groups; dichlorophenoxyacetic acid; 2,4-d; cesium; chlorine and chlorine compounds; chlorine compounds; chlorine compounds; chlorine gas; chlorine, ionic species; chlorides; chlorine, ionic species; perchlorates; chromium; chromium-containing ionic species; chromate ion, hexavalent chromium; cobalt; copper; cyclic ethers; cyclic ethers; dioxane-1,4; ethylene oxide; dihalogenated and polyhalogenated ethers; dichloroethyl, 1,1-ether; dichloromethyl, 1,1-ether; methyl-dichloro-1,1-ether; dieldrin; dihalogenated and polyhalogenated ethers; endrin; trichlorophenoxyacetic, 2,4,5 acid; dioxin and related compounds; dioxin and related compounds; hxcdf; hexachlorodibenzofurans; tcdd; tetrachlorodibenzodioxins; tcdf; tetrachlorodibenzofurans; disubstituted and polysubstituted benzene hydrocarbons; diethyl benzene; disubstituted and polysubstituted benzene hydrocarbons; ethyltoluene; indene; dihydroindene; trimethyl benzene; xylenes; elemental sulfur; epoxides; esters; butyl acrylate; propenoic acid, butyl ester; esters; ethyl acetate; ethyl acrylate; ethyl lactate; hydroxypropanoic acid, ethyl ester; methyl methacrylate; phthalate, butyl benzyl; butyl benzyl phthalate; phthalate, dibutyl; dibutyl phthalate, *n*; phthalate, diethylhexyl; diethylhexyl phthalate; phthalate, dimethyl; dimethyl phthalate; phthalate, dioctyl; di-*n*-octyl phthalate; vinyl acetate; ethers; fluoride ion; glycols; ethylene glycol; glycols; propylene glycol; propylene glycol monoethyl ether acetate (pgmea); glycols, epoxides; gold; halogenated aromatic compounds; halogenated cresols; halogenated ethers and epoxides; halogenated phenolic compounds; halophenols; chlorophenol; dichlorophenol; halophenols; pentachlorophenol; pcp; tetrachlorophenol; trichlorophenol; heterocyclic nitrogen compounds; heterocyclic oxygen compounds; heterocyclic oxygen compounds with three or more rings; dibenzofuran; oxygen heterocycles with three or more rings; hydrazine derivatives; dimethylhydrazine, *n,n;* unsymmetrical dimethylhydrazine; hydrazine derivatives; methylhydrazine; monomethylhydrazine; iodine; ionic species containing iron; ferrocyanide ion; ions containing phosphorus; phosphate; ions containing sulfur; sulfate ion; sulfide ion; ions with nitrogen; ammonium ion; nh4+; cyanide *(*cn-*)*; hydrazine; nitrate *(*no3-*)*; iron; ketones; acetone; butanone; mek; methyl ethyl ketone; cyclohexanone; isophorone; isopropylacetone; methyl isobutyl ketone; hexone; 4-methyl-2-pentanone; ketones; lanthanides; lead; lead compounds; lead compounds; tetraethyl lead; lithium; magnesium; manganese; mercury; molybdenum; monohydric phenols; monohydric phenols; phenol; phenol, dimethyl; xylenols; phenol, methyl; cresols; methyl phenol; nickel; nitrogen compounds; ammonia; nitrogen trifluoride; nitrophenolic compounds; nitrophenols; nitrophenol, 4; nitrophenol, *p*; nitrophenols; picric acid; trinitrophenol; nitrosamines; noncyclic aliphatic or aromatic ethers; diethyl ether; methyl *tert-*butyl ether; mtbe; methyl tertiary butyl ether; noncyclic aliphatic or aromatic ethers; tetrahydrofuran; organophosphonates; diisopropyl methyl phosphonate; dimp; organophosphonates; organophosphorus compounds; organosulfur compounds with other functional groups; endosulfan; organosulfur compounds with other functional groups; other nitrophenols; dinoseb; *sec*-butyl dinitrophenol; dinitrophenol, 4,6-*sec*-butyl; other nitrophenols; pesticides; pesticides/herbicides; phenols; platinum; plutonium; polycyclic aromatic hydrocarbons with more than five fused rings; benzo(*g*,*h*,*i*)perylene; polycyclic aromatic hydrocarbons with more than five fused rings; polycyclic aromatic hydrocarbons, four-ring compounds; benzo(*a*)anthracene; chrysene; polycyclic aromatic hydrocarbons with four fused rings; pyrene; polycyclic aromatic hydrocarbons, two- or three-ring compounds; acenaphthene; acenaphthylene; anthracene; methylnaphthalene; naphthalene; phenanthrene; polycyclic aromatic hydrocarbons with two or three fused rings; polycyclic aromatic hydrocarbons; pah; pna; pom; polycyclic hydrocarbons, nonalternant compounds with four fused rings; fluoranthene; four-ring fused nonalternant hydrocarbons; polycyclic hydrocarbons, nonalternant compounds with five fused rings; benzo(*b*)fluoranthene; benzo(*k*)fluoranthene; polycyclic hydrocarbons, nonalternant compounds with five fused rings; polycyclic hydrocarbons, nonalternant compounds with fused rings; polycyclic hydrocarbons, nonalternant compounds with more than five fused rings; indeno(1,2,3-*cd*)pyrene; polycyclic hydrocarbons, nonalternant compounds with more than five fused rings; polycyclic hydrocarbons, nonalternant compounds with two or three fused rings; fluorene; indene; polycyclic hydrocarbons, nonalternant compounds with two or three fused rings; polycyclic aromatic hydrocarbons, five-ring compounds; benzo(*a*)pyrene; dibenzo(*a*,*h*)anthracene; polycyclic aromatic hydrocarbons, four-ring compounds; potassium; primary alcohols; benzyl alcohol; butanol, *n*; ethanol; methanol; primary alcohols; primary aliphatic amines and diamines; hexylamine; monomethylamine; primary aliphatic amines; pyridine and substituted pyridines; paraquat; pentachloropyridine; pyridine; pyridine and substituted pyridines; pyrrole and fused-ring derivatives of pyrrole; captan; pyrrole and fused-ring derivatives of pyrrole; radionuclides; radium; ring-substituted aromatics; chlorobenzene; monochlorobenzene; chlorotoluene, 2; ddd; dichloro(chlorophenyl)-bis ethane; dde; dichlorodiphenyldichloroethylene; dichlorobenzene, 1,2; dichlorobenzene, 1,3; dichlorobenzene, 1,4; hexachlorobenzene; polychlorinated benzenes; polychlorinated biphenyls; pcbs; aroclor; ring-substituted aromatics; tetrachlorobenzene; trichlorobenzene, 1,2,4; saturated alkyl halides; bromodichloromethane; bromoform; tribromomethane; butyl chloride; chlorobutane; carbon tetrachloride; carbon tetrafluoride; chloroform; trichloromethane; chloromethane; methyl chloride; dibromochloromethane; dibromoethane, 1,2; ethylene dibromide; dibromomethane; dichlorodifluoromethane; freon 12; dichloroethane, 1,1; dichloroethane, 1,2; dca; dichloropropane, 1,2; dichlorotrifluoroethane; hcfc-1,2,3; ethyl chloride; chloroethane; freon 111; freon 113; hexachlorocyclohexane; lindane; hexachloroethane; hexafluoroethane; methyl bromide; bromomethane; methylene chloride; dichloromethane; saturated alkyl halides; tetrachloroethane, 1,1,2,2; trichloroethane, 1,1,2; trichloroethane, 1,1,1; tca; trichlorofluoromethane; freon 11; fluorocarbon 11; trichloropropane, 1,2,3; secondary alcohols; isobutyl alcohol; propanol, 2; isopropyl alcohol; isopropanol; secondary alcohols; secondary aliphatic amines; hmx, octagen, octahydro-1,3,5,7-tetranitro-1,3,5,7-tetrazine; rdx, cyclonite, hexahydro-1,3,5-trinitro-1,3,5-triazine; secondary aliphatic amines; selenium; silicon compounds-other significant; hexamethyldisilicon; silver; simple aromatic nitro compounds; dinitrotoluene; nitrobenzene; simple aromatic nitro compounds; trinitrotoluene; tnt; single-ring heterocyclic sulfur compounds; single-ring heterocyclic sulfur compounds; tetrachlorothiophene; sodium; strontium; sulfides, disulfides; dimethyl disulfide; dimethyl sulfide; sulfides, disulfides; sulfonic acids; sulfonic acids, sulfoxides; sulfoxides; sulfur, compounds and ions; sulfur compounds; carbon disulfide; hydrogen sulfide; sulfur oxides; sulfur compounds-other significant; sulfur hexafluoride; technetium; tertiary alcohols; tertiary amines (alkyl, aryl); tertiary amines (alkyl, aryl); thallium; thiols; methanethiol; methyl mercaptan; thiols, mercaptans; thiols, sulfides, and disulfides; thorium; tin; titanium; total petroleum hydrocarbons (tph); tungsten; unsaturated alkyl halides; aldrin; chlordane; octachlorohexahydromethanoindene; dichloroethene, 1,1; dichloroethene, 1,2; dce; heptachlor; hexachlorobutadiene; hexachlorocyclopentadiene; tetrachloroethene; perchloroethylene; pce; toxaphene; chlorinated camphor; trichloroethene; trichloroethylene; tce; unsaturated alkyl halides; vinyl chloride; uranium; vanadium; volatile organic compounds (vocs); zinc; and zirconium

### TRPC4 dominant negative polypeptides and antibody targets

In order to find out the essential domain for pore formation Schind et al. Cell Calcium 43 (2008) 260-269 created dominant negative N-term fragments corresponding to N-terms that contain the domains stated above. The only dominant negative fragment required to successfully suppress TRPC4 currents was the first ankyrin domain (Table 49).

The rat domain is designated: 61-ININCIDPLG RTALLIAIEN ENLELIELLL SFNVYVGDAL LHAIRKEVVG AVELLLNHKK-121

The mouse domain is designated: 61-ININCIDPLG RTALLIAIEN ENLELIELLL SFNVYVGDAL LHAIRKEVVG AVELLLNHKK-121

The *Trpc4* N-terminal, and more specifically the ankyrin repeat motifs, and more specifically the first ankyrin domain have been found to be essential for Trpc4 pore formation. Therefore, *Trpc4* inhibition for the treatment of pain involves antibodies such as but not limited to targeting the N-terminal, ankryin repeats, first ankyrin repeat, all extracellular domains, and all cytoplasmic domains.

### G protein derived modulators of TRPC4

TRPC4 has properties similar to Ca2+-permeable nonselective cation channels (NSCCs) which are activated by muscarinic stimulation and can be blocked by anti Gα/o antibodies (G-protein). The specificity of G protein activation of *Trpc4* was studied by Jeon et al Bioch. Biophy. Res. Comm. 377 (2008) 538. The group transfected a *Trpc4* vector into human embryonic kidney (HEK293) cells, *Trpc5* and *Trpc6* were also transfected, the cells were trypsinized and used for whole cell recordings. In order to obtain a current-voltage (I-V) relationship GTPyS which activates *Trpc4* is added to the intracellular solution. Constitutively activated G protein mutants (Gα) were expressed in the HEK cells. Only Gai2 was able to stimulate TRPC4 above the mock level without GTPyS. Gaq and Gα11 decreased current with and without GTPyS stimulation. When a Gai2 was expressed without *Trpc4* it did not activate I-V TRRPC like currents (Figure 58). No potentiation activity was observed for *Trpc5* currents with Gai2 expression (Figure 59). Therefore, this is strong evidence that Gai2 specifically activates *Trpc4* since it shares 80% N-terminal homology with *Trpc5.* Gaq inhibits TRPC5 currents as well as TRPC4 currents; studies were not recorded with Gα11 (Figure 59). These studies indicate that expression of G protein Gai2 has the ability to specifically activate *Trpc5* and expression of G protein Gaq and Gα11have the ability to inhibit both *Trpc4* and *Trpc5.*

G protein derivatives have been shown to inhibit *Trpc4.* Therefore, *Trpc4* inhibition for the treatment of pain involves but is not limited to the use of G protein analogs, peptides, dominant negative mutants such as but not limited to the following: Gaq, Gai2

### TRPC4 inhibiting ions

Trivalent ion lanthanum derivatives are known to inhibit TRP channels. Therefore, *Trpc4* inhibition for the treatment of pain involves the use of trivalent ion lanthanum analogs.

### TRPC4 inhibiting RNA

Specific short interfering RNAs (siRNAs) against TRPC4. siRNAs have been shown to inhibit TRPC4 function in multiple domains. Therefore, *Trpc4* inhibition for the treatment of pain involves the use of RNA analogs.

Trpc*4* activator targeted polypeptides have been shown to inhibit TRP channels. Therefore, *Trpc4* activator polypeptides, including but not limited to peptidomimetics (e.g. by peptide phage display), which bind and block the receptor without activation, and peptide analogs, inhibit *Trpc4* and can be used for the treatment of pain.

### Tables

**Table 1**

| **KO** | **WT (M)** | **KO (F)** | **WT (F)** |
|---|---|---|---|
| **4.2** | **4.1** | **5.5*** | **4.3** |
| **±0.07** | **±0.15** | **±0.31** | **±0.12** |

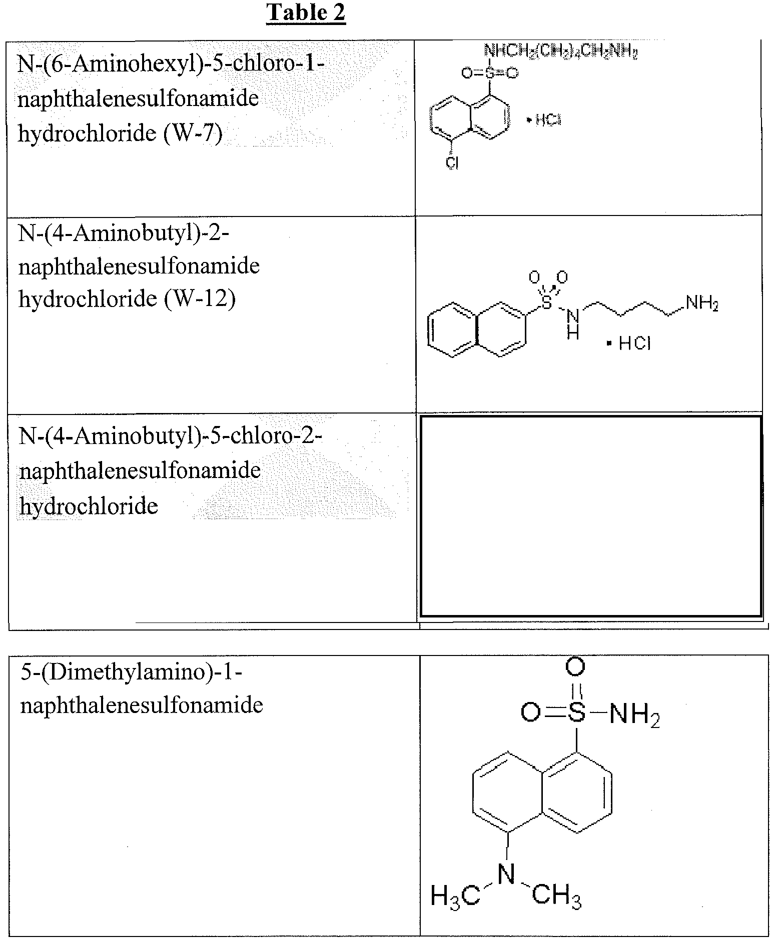

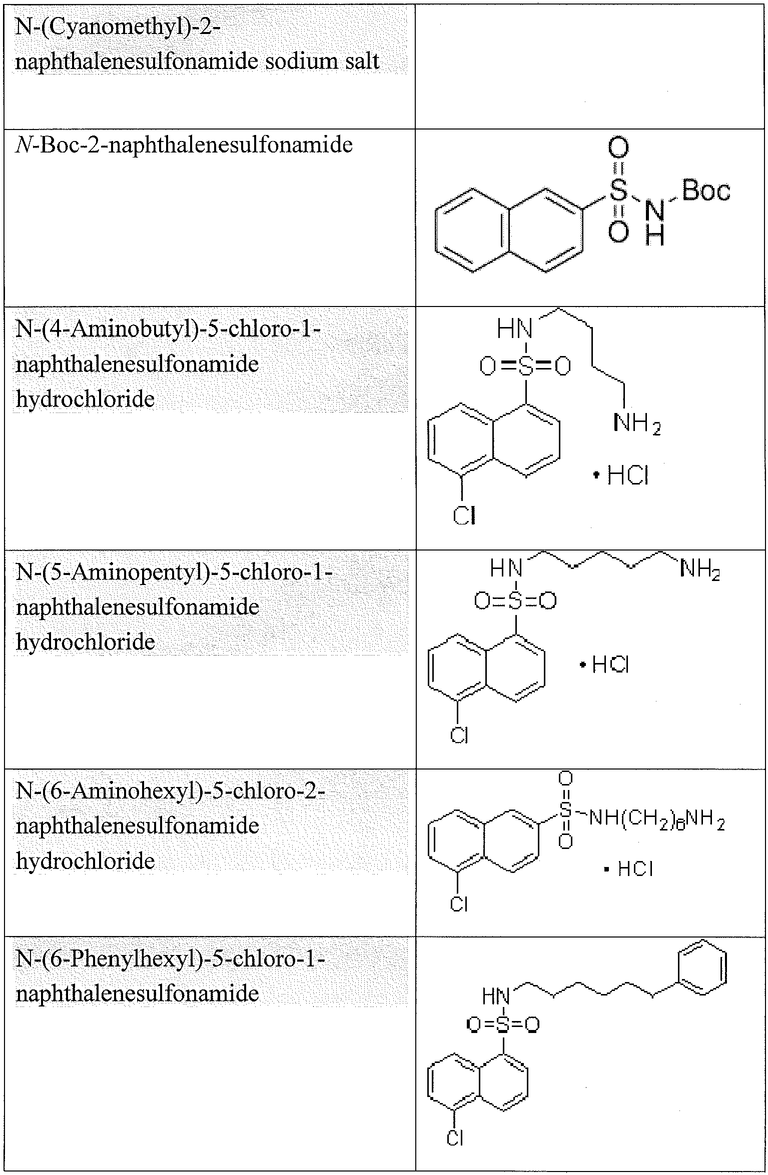

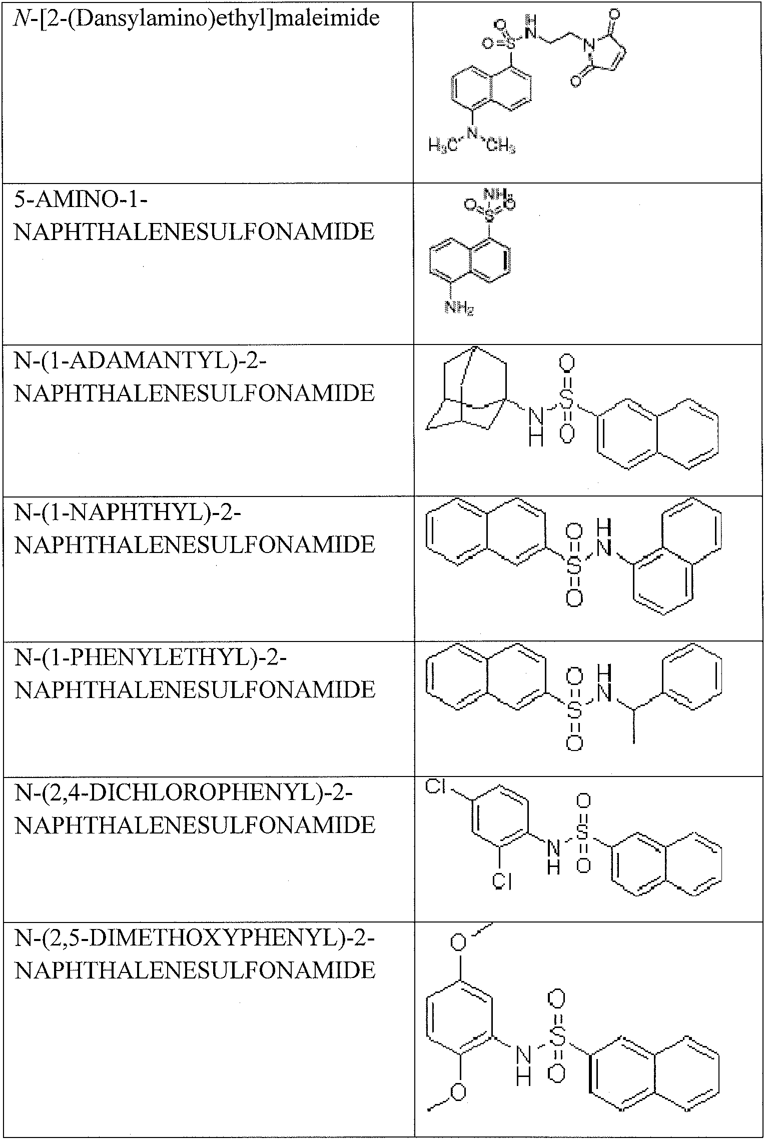

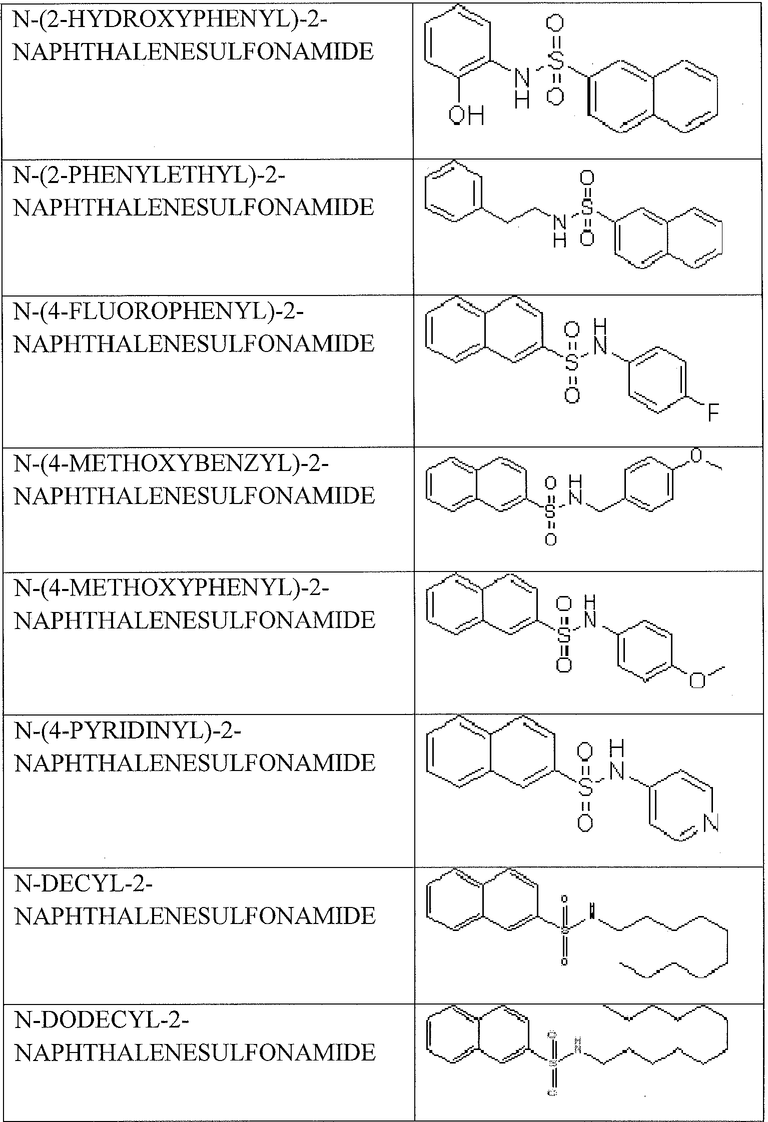

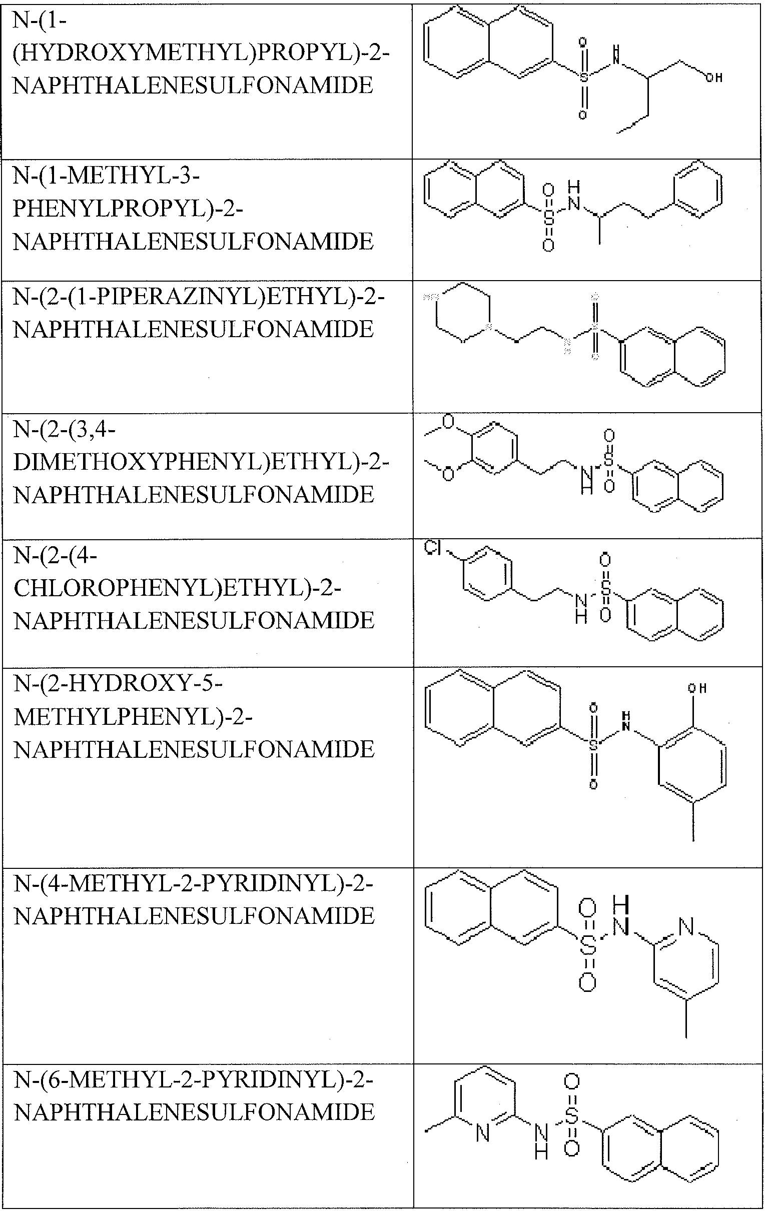

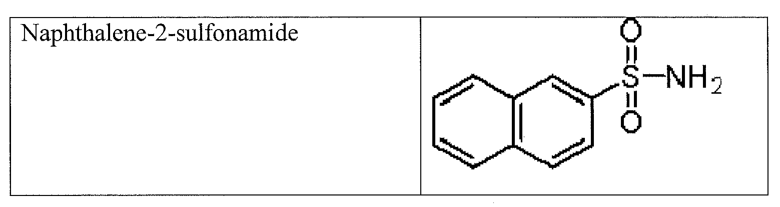

**Table 3**

| | |
|---|---|
| Trifluoperazine dihydrochloride | 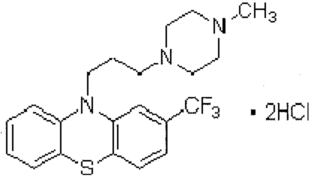 |

Phenothiazine, 2-(Trifluoromethyl)phenothiazine, Chlorpromazine hydrochloride, Ethopropazine hydrochloride, Promazine hydrochloride, Promethazine hydrochloride, Promethazine-d₄ hydrochloride, Thioridazine hydrochloride, 2-Methylthiophenothiazine, Promethazine-d₇ hydrochloride, Mesoridazine benzenesulfonate.

**Table 4**

| | |
|---|---|
| F endiline | 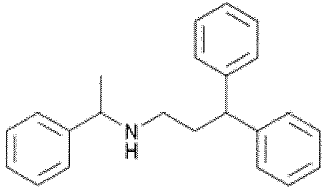 |

**Table 5**

| | |
|---|---|
| Vincristine sulfate | 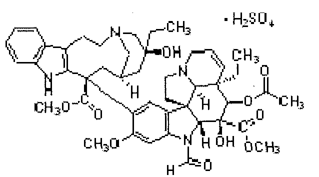 |
| Vinblastine | 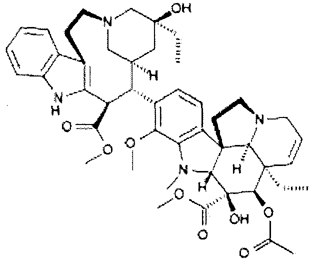 |
| KAR-2 | 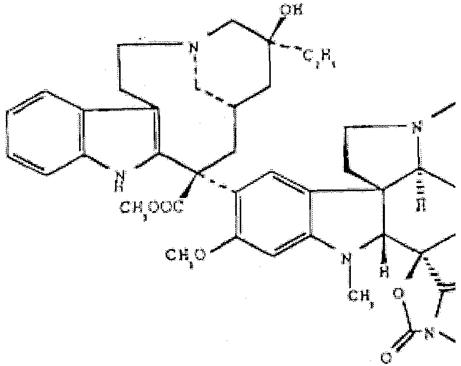 |
| | KAR-2 |

**Table 6**

| | |
|---|---|
| Malbrancheamide | 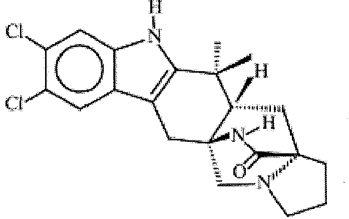 |
| Piperine | 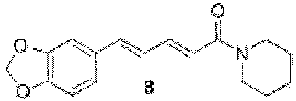 |

**Table 7**

| | |
|---|---|
| tajixanthone hydrate | 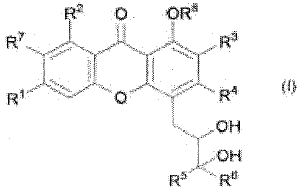 |

**Table 8**

| | |
|---|---|
| Autocamtide 2-related inhibitory peptide | Lys-Lys-Ala-Leu-Arg-Arg-Gln-Glu-Ala-Val- Asp-Ala-Leu |
| Neuropeptide Y Fragment 22-36 | Ser-Ala-Leu-Arg-His-Tyr-Ile-Asn-Leu-Ile- Thr-Arg-Gln-Arg-Tyr-NH2 |
| Melittin | GIGAVLKVLTTGLPALISWIKRKRQQ |

**Table 9**

| | |
|---|---|
| Phenoxybenzamine hydrochloride | 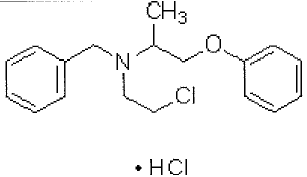 |

**Table 10**

| | |
|---|---|
| 8-Methoxymethyl-3-isobutyl-1-methylxanthine | 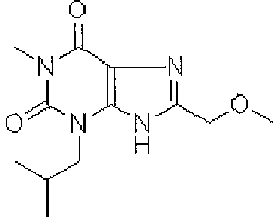 |

**Table 11**

| | |
|---|---|
| D-Sphingosine | 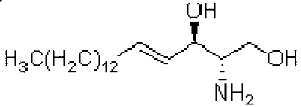 |

**Table 12**

| | |
|---|---|
| KN-93; N-[2-[N-(4-Chlorocinnamyl)-N-methylaminomethyl]phenyl]-N-(2-hydroxyethyl)-4-methoxybenzenesulfonamide phosphate salt, N-[2-[[[3-(4'-Chlorophenyl)-2-propenyl]methylamino]methyl]phenyl]-N-(2-hydroxyethyl)-4'-methoxybenzenesulfonamide phosphate salt | 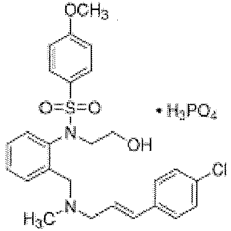 |

**Table 13**

| | |
|---|---|
| Capsazepine | 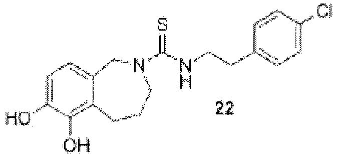 |

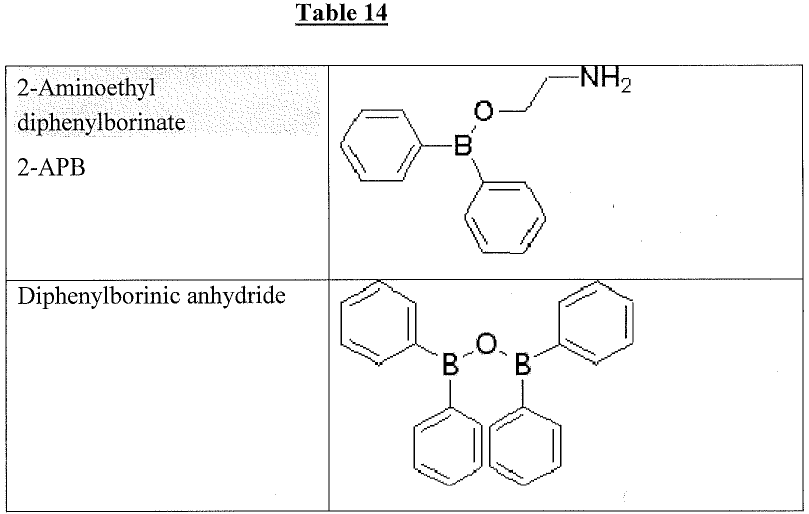

**Table 15**

| | |
|---|---|
| Phorbol 12-myristate 13-acetate (PMA) | 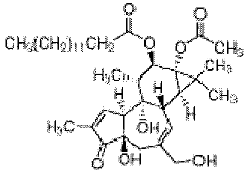 |
| Phorbol 12,13-diacetate | 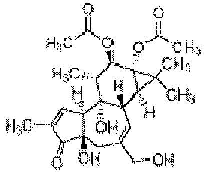 |
| Phorbol 12,13-dibutyrate | 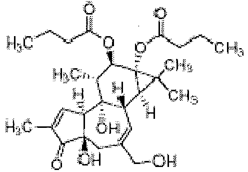 |

**Table 16**

| | |
|---|---|
| N-(3-Methoxyphenyl)-4-chlorocinnamide | 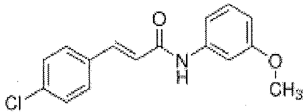 |
| α-Cyano-(3-ethoxy-4-hydroxy-5-phenylthiomethyl)cinnamide | 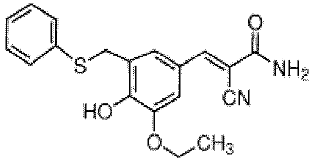 |
| (*E*)-3-(4-*t*-butylphenyl)-*N-*(2,3-dihydrobenzo[*b*][1,4]dioxin-6-yl)acrylamide | 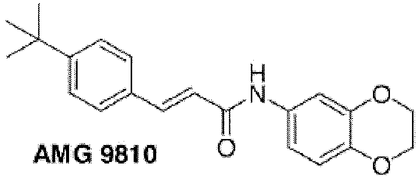 |
| AMG0347 | 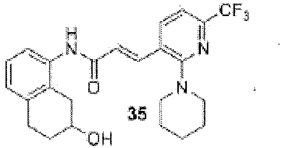 |

**Table 17**

| | |
|---|---|
| 5'-Iodoresiniferatoxin | 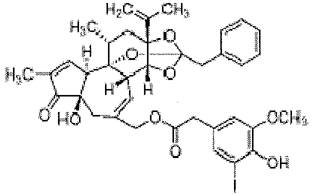 |
| 6,7-Deepoxy-6,7-didehydro-5-deoxy-21-dephenyl-21-(phenylmethyl)-20-(4-hydroxybenzeneacetate)daphnetoxin | 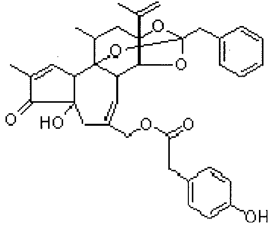 |
| 5-iodoRTX | 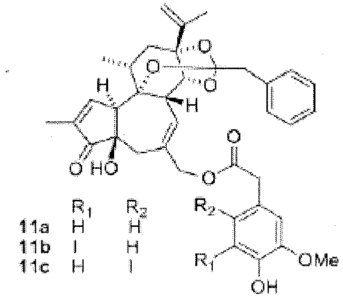 |

**Table 18**

| | |
|---|---|
| *N*-(4-tertiarybutylphenyl)-4-(3-chloropyridin-2-yl)tetrahydropyrazine-1(2*H*)-carbox-amide (BCTC) | 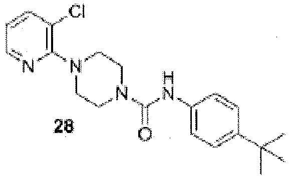 |
| 2-Methyl-3-(methylthio)pyrazine | 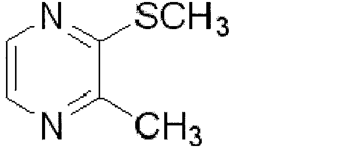 |
| 5*H*-5-Methyl-6,7-dihydrocyclopenta[*b*]pyrazine | 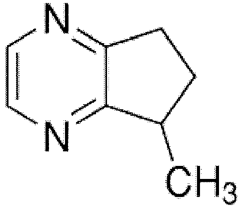 |

**Table 19**

| | |
|---|---|
| 1-Isoquinolin-5-yl-3-(4-trifluoromethyl-benzyl)-urea | |
| ISOQUINOLIN-1-YL-PYRIDIN-3-YLMETHYL-AMINE, DIHYDROBROMIDE | 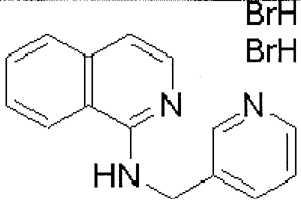 |

**Table 20**

| | |
|---|---|
| (1R, 2S, 5R)-N-(4-methoxyphenyl)-5-methyl-2-(1-methylethy I)cyclohexanecarboxamide | 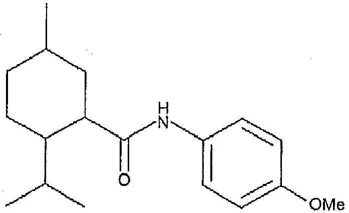 |
| N-(1-PHENYLETHYL)CYCLOHEXAN ECARBOXAMIDE | 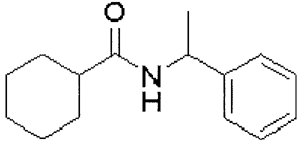 |

**Table 21**

| | |
|---|---|
| Thapsigargi n | 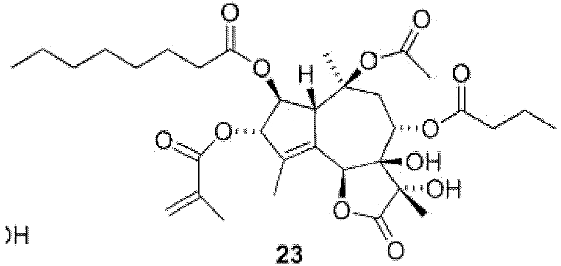 |
| Parthenolide | 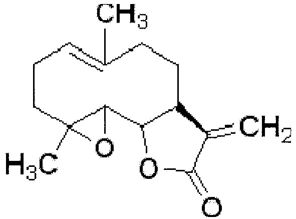 |

**Table 22**

| | |
|---|---|
| Yohimbine | 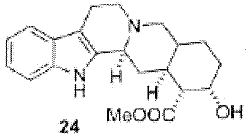 |
| α-Yohimbine hydrochloride, 17α-Hydroxy-20α-yohimban-16 β-carboxylic acid methyl ester hydrochloride | 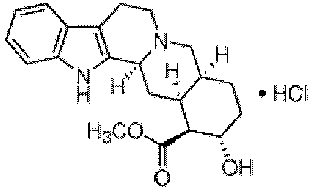 |

**Table 23**

| | |
|---|---|
| Cannabidiol | 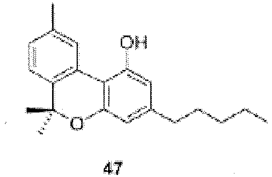 |

**Table 24**

| | |
|---|---|
| AMP | 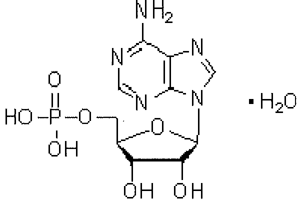 |
| (-)-Adenosine 3'-monophosphate hydrate | 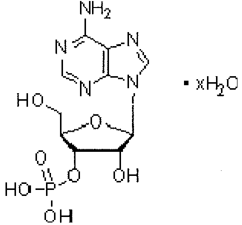 |

**Table 25**

| | |
|---|---|
| spermine | 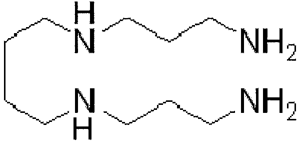 |
| 1-Naphthylacetyl spermine trihydrochloride | 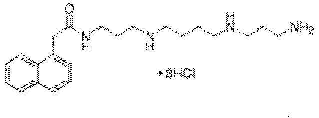 |

**Table 26**

| | |
|---|---|
| 10-Camphorsulfonyl chloride | 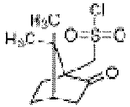 |
| camphor | 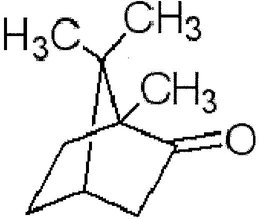 |

**Table 27**

| | |
|---|---|
| ethanol | CH₃CH₂OH |
| 2-(2-Chloroethoxy)ethanol | 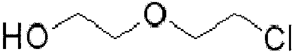 |

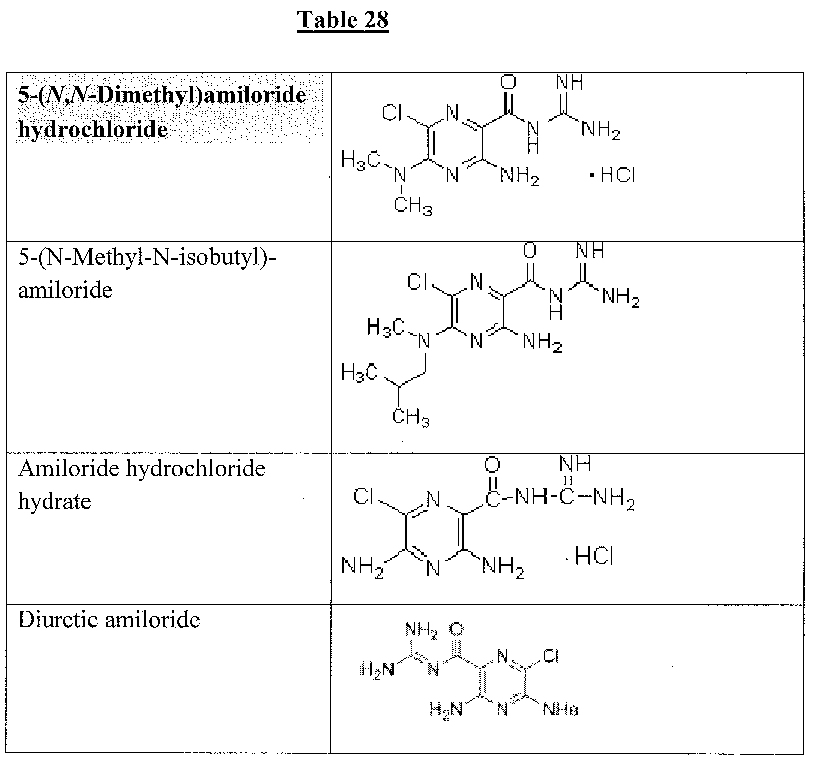

**Table 29**

| | |
|---|---|
| Econazole nitrate | 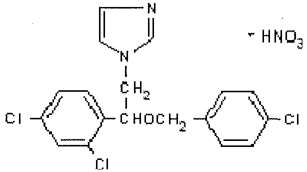 |
| 1-(Trimethylsilyl)imidazole | 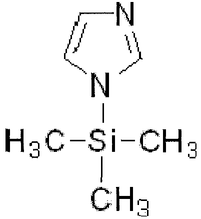 |
| Clotrimazole | 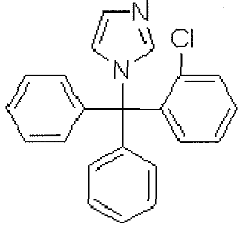 |
| 1-[4-(Trifluoromethyl)phenyl]imidazole | 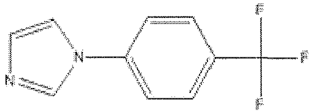 |
| Azole econazole | 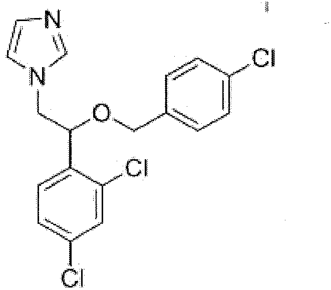 |

**Table 30**

| | |
|---|---|
| Verapamil hydrochloride | 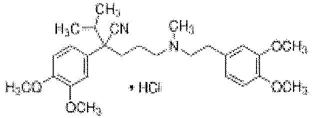 |

**Table 31**

| | |
|---|---|
| A-425619 | 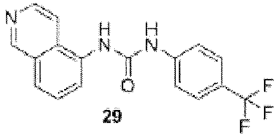 |
| SB-452533 | 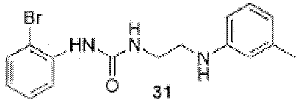 |
| ABT-102 | 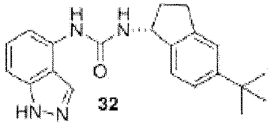 |
| JYL1421 | 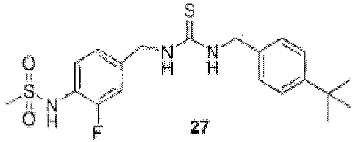 |
| SB-366791 | 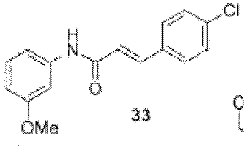 |
| 4-(α-pyridyl)piperidine-1-acyl chlorides | |
| Phenylacetylrinvanil (PhAR) | |

**Table 32**

| | |
|---|---|
| AMG517 | 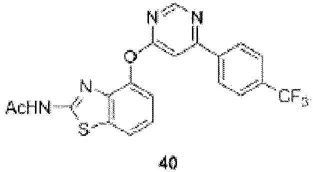 |

**Table 33**

| | |
|---|---|
| SB-782443 | 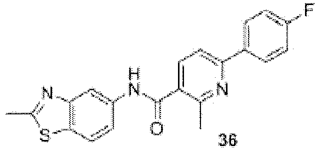 |
| Thiazolycarboxamide | 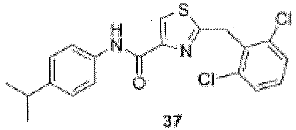 |
| Tetrahydropyridylcarboxamide | 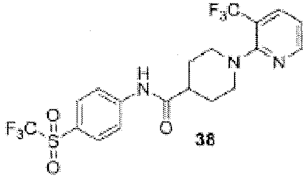 |

**Table 34**

| | |
|---|---|
| 4-aminopyridine | 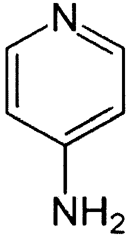 |

**Table 35**

| | |
|---|---|
| Tetraethylammonium | 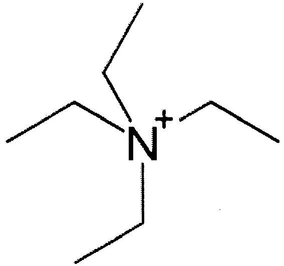 |

**Table 36**

| | |
|---|---|
| Monoterpene aldehyde citral | 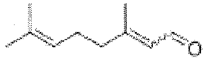 |

**Table 37**

| | |
|---|---|
| Arsane | 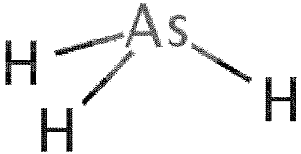 |
| RN-1734; 1-alkoxycarbonylalkylidenetriphenylarsoranes | |
| RN-9893 | |

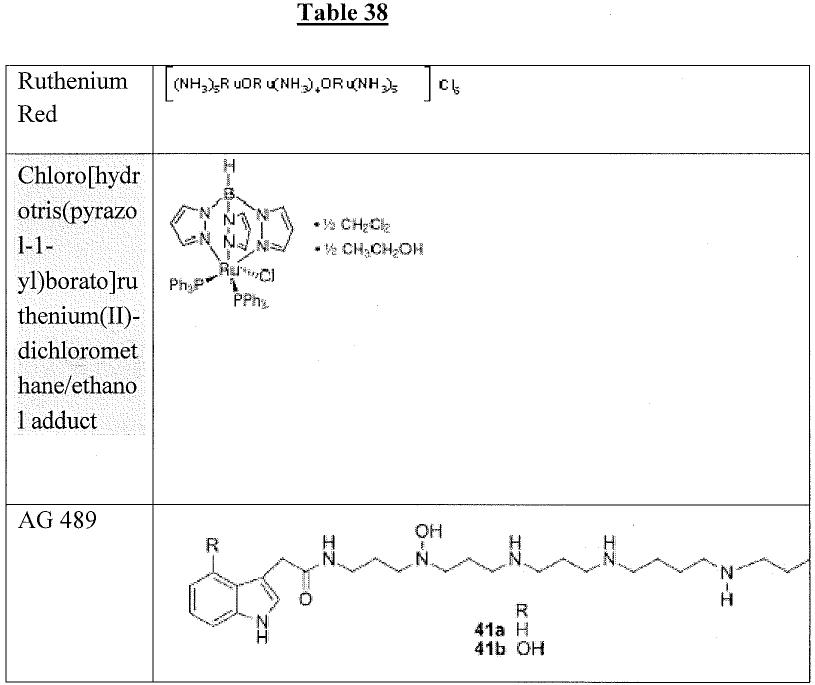

**Table 39**

| | |
|---|---|
| *N-*arachidonylethanolamine (AEA, Anandamide) | 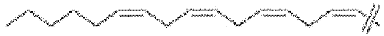 |
| *N-*arachidonoyldopamine (NADA) | 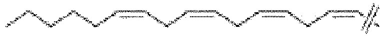 |
| *N*-oleoylethanolamine (OLEA) | 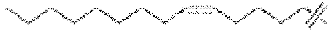 |

**Table 40**

| | |
|---|---|
| 5-lipoxygenase | 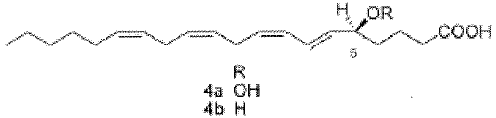 |
| 12-lipoxygenase | 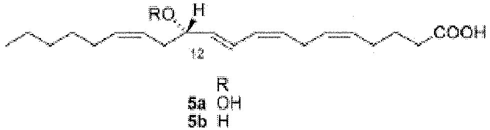 |
| 15-lipoxygenase | 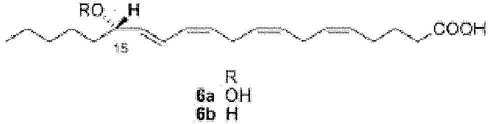 |

**Table 41**

| | |
|---|---|
| Capsaicinoids (a), capsinoids (b) | 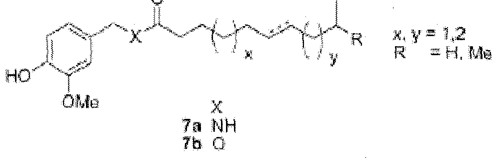 |
| Oleoylvanillamine (a), phenylacetylrinvanil (b) | 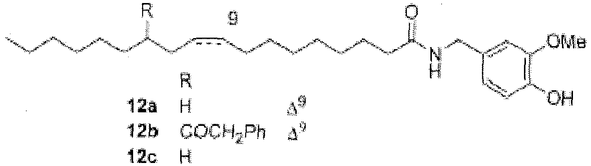 |
| SDZ-249482 (a), SDZ-249665 (b) | 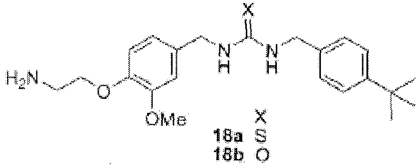 |
| Capsavanil | 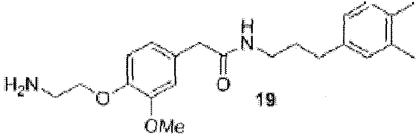 |
| zucapsaicin | 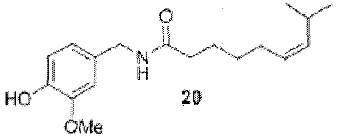 |

**Table 42**

| | |
|---|---|
| Eugenol | 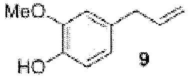 |

**Table 43**

| | |
|---|---|
| Lidocaine | 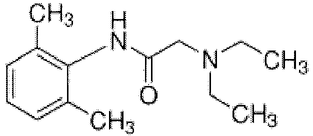 |

**Table 44**

| | |
|---|---|
| Allicin | 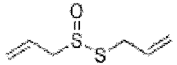 |
| Alliin | 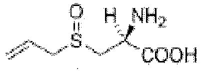 |

**Table 45**

| | |
|---|---|
| 12-deoxyphorbol 13-isobutyrate 20-acetate (DPBA) | |

**Table 46**

| | |
|---|---|
| Probenecid | 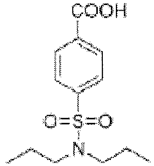 |

**Table 47**

| | |
|---|---|
| Cannabidiol | 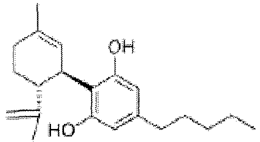 |
| Tetrahydrocannabinol | 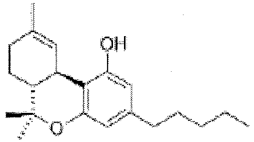 |
| Cannabinol | 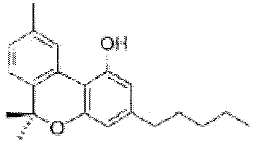 |

**Table 48**

| | |
|---|---|
| Incensole acetate | 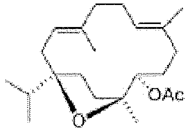 |

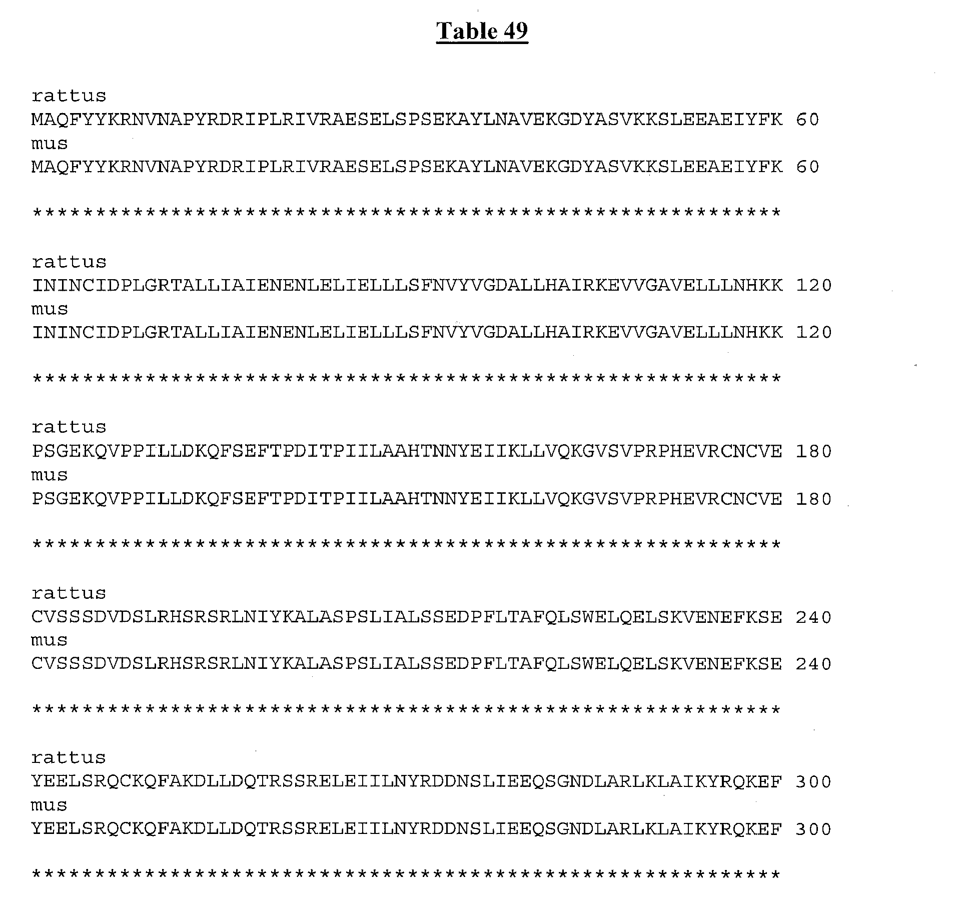

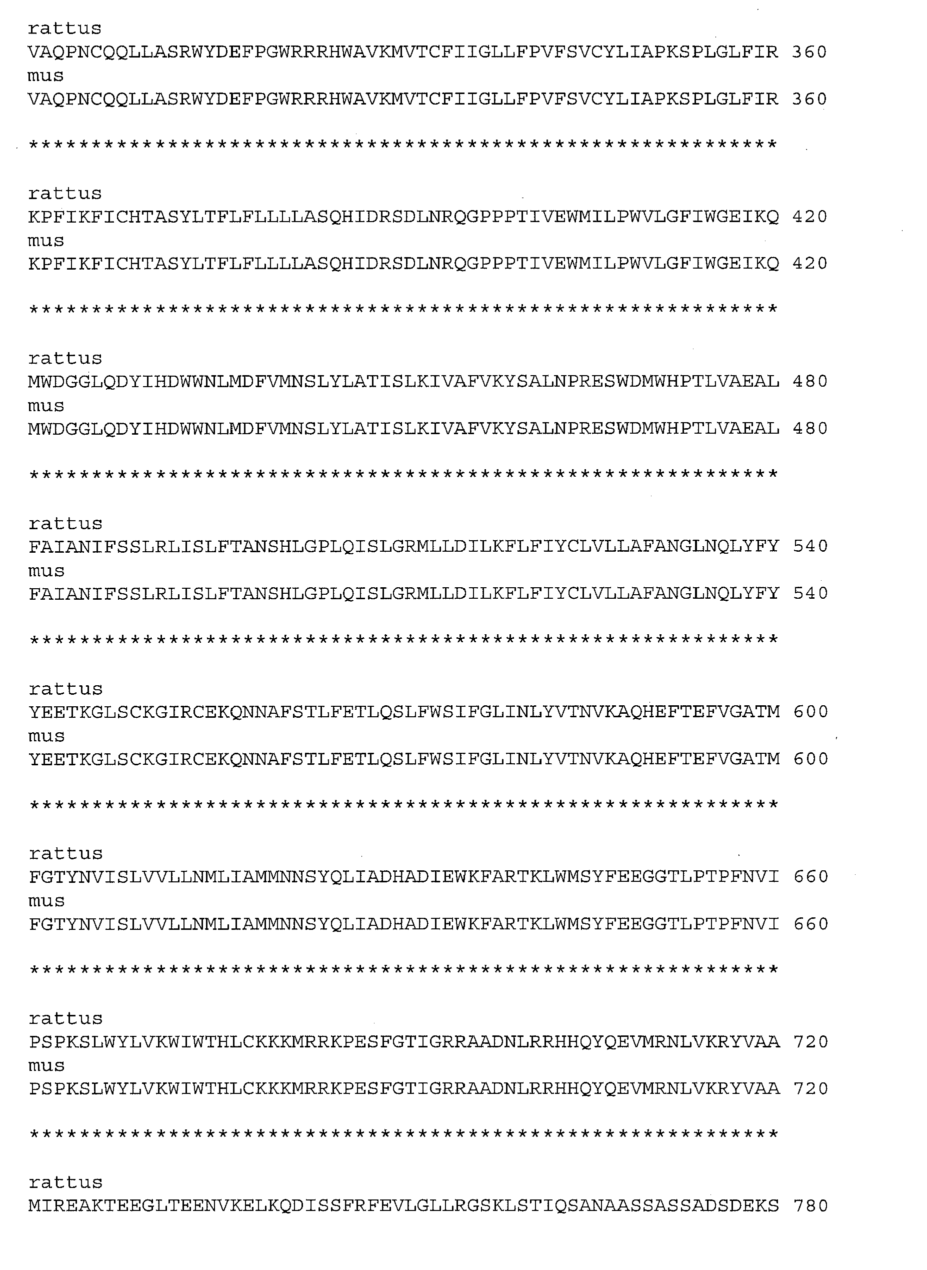

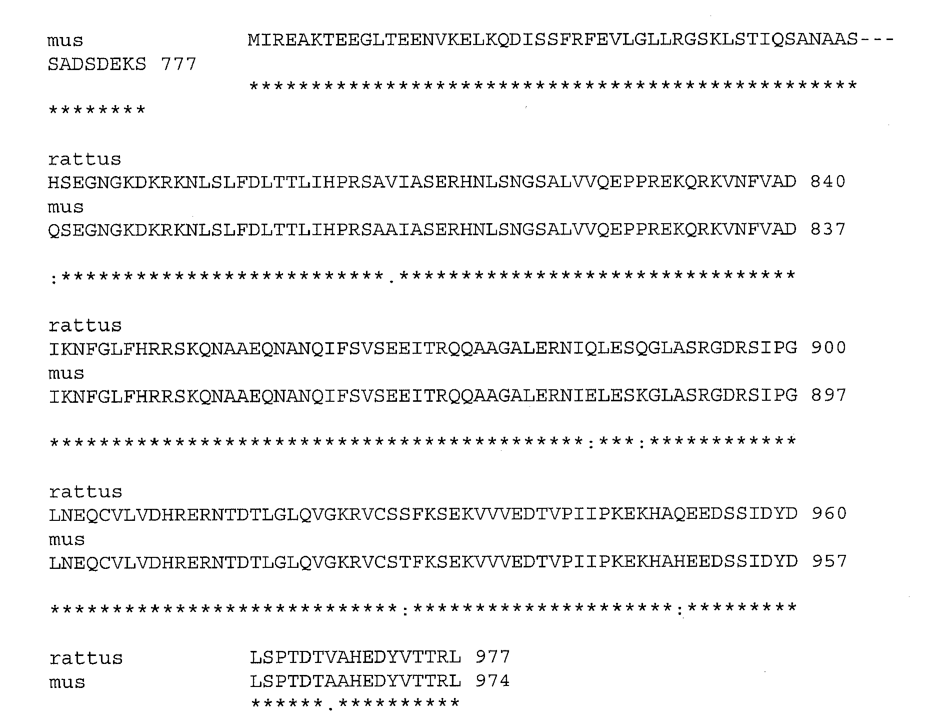

**Table 50**

| |
|---|
| Metabotropic glutamate receptors (mGluR) |
| Gq/11 protein coupled receptors |
| Brain-derived neurotrophic factor (BDNF) |
| Phospholipase C and inhibitor U73122 |
| Phosphatidylinositol-3-OH kinase (PI3K) and inhibitor LY294002 |
| PLC activator m-3M3FBS |
| Downstream activator of PLC, inositol-1,4,5-trisphosphate (InsP3) |
| Diacylglycerol (DAG)-protein kinase C |
| PKC specific inhibitors GF109203X and Go6976 |

## Claims

1. A compound, selected from 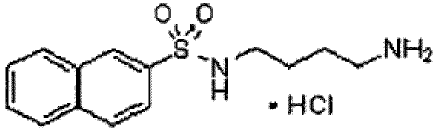
N-(4-(Aminobutyl)-2-naphthalenesulfonamide hydrochloride (W12); and
N-(4-(Aminobutyl)-5-chloro-2-naphthalenesulfonamide hydrochloride (W13)
for use in treating neuropathic pain by inhibiting TRPC4 activity.

2. The compound for the use of claim 1, wherein the pain is chronic.

3. The compound for the use of claim 2, wherein the pain is associated with cancer.

4. The compound for the use of claim 1, wherein the pain is acute.

5. The compound for the use of claim 4, wherein the pain is associated with a soft tissue injury, an infection or an inflammation.

## Patentansprüche

1. Verbindung, ausgewählt aus: 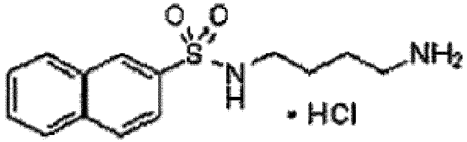
N-(4-(Aminobutyl)-2-naphthalinsulfonamid-hydrochlorid (W12); und
N-(4-(Aminobutyl)-5-chlor-2-naphthalinsulfonamidhydrochlorid (W13)
zur Verwendung bei der Behandlung eines neuropathischen Schmerzes durch Inhibition der TRPC4-Aktivität.

2. Verbindung zur Verwendung nach Anspruch 1, wobei der Schmerz chronisch ist.

3. Verbindung zur Verwendung nach Anspruch 2, wobei der Schmerz mit Krebs assoziiert ist.

4. Verbindung zur Verwendung nach Anspruch 1, wobei der Schmerz akut ist.

5. Verbindung zur Verwendung nach Anspruch 4, wobei der Schmerz mit einer Weichteilverletzung, einer Infektion oder einer Entzündung assoziiert ist.

## Revendications

1. Composé choisi parmi 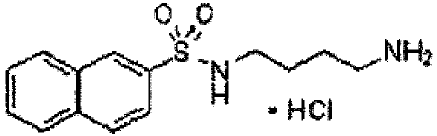
Chlorhydrate de N-(4-(aminobutyl)-2-naphtalènesulfonamide (W12) ; et
Chlorhydrate de N-(4-(aminobutyl)-5-chloro-2-naphtalènesulfonamide (W13)
destiné à être utilisé pour le traitement de la douleur neuropathique par inhibition de l'activité des TRPC4.

2. Composé destiné à être utilisé selon la revendication 1, dans lequel la douleur est chronique.

3. Composé destiné à être utilisé selon la revendication 2, dans lequel la douleur est associée au cancer.

4. Composé destiné à être utilisé selon la revendication 1, dans lequel la douleur est aiguë.

5. Composé destiné à être utilisé selon la revendication 4, dans lequel la douleur est associée à une lésion des tissus mous, une infection ou une inflammation.
